# EUROPÄISCHE PATENTSCHRIFT

(11) **EP 0 600 218 B1**
(45) Veröffentlichungstag und Bekanntmachung des Hinweises auf die Patenterteilung: **01.04.1998**
(21) Anmeldenummer: 93117295.1
(22) Anmeldetag: 26.10.1993
(51) Int. Cl.: A61F 13/06, A61F 5/01

(54) **Bandage für das Kniegelenk**
Bandage for the knee joint
Bandage pour l'articulation du genou

(30) Priorität: 05.11.1992 DE 4237389; 04.08.1993 EP 93112490
(43) Veröffentlichungstag der Anmeldung: 08.06.1994
(73) Patentinhaber: Beiersdorf Aktiengesellschaft, 20245 Hamburg (DE)
(72) Erfinder: Brandt, Dieter, D-40545 Düsseldorf (DE); Szlema, Ingeborg, D-47906 Kempen (DE)
(74) Vertreter: Richter, Werdermann & Gerbaulet

(56) Entgegenhaltungen:
- EP-A- 0 154 758
- EP-A- 0 496 071
- EP-A- 0 498 062
- WO-A-85/04569
- DE-A- 4 103 383
- FR-A- 2 607 384
- FR-A- 2 636 228
- US-A- 4 116 236
- US-A- 4 287 885
- US-A- 4 986 263

## Beschreibung

Die Erfindung bezieht sich im wesentlichen auf eine Bandage für das Kniegelenk aus elastischem Bandagenstoff in Schlauchform mit einem vorderen Bandagenteil und einem rückwärtigen Bandagenteil und mit mindestens einem eingearbeiteten, längsverlaufenden Federstab, wobei die Bandage aus einem anatomisch geformten Schlauchkörper aus einem Gewebe besteht und wobei in dem vorderen Bandagenteil eine im Bereich über der Kniescheibe bei angelegter Bandage liegende, die Quadrizeps-Sehne freilassende Pelotte aus einem weichen oder weichelastischen Material angeordnet ist (vergl. Oberbegriff der Ansprüche 1 bis 5).

Das Knie ist das verletzungsgefährdetste und am häufigsten von Arthrosen betroffene Gelenk des Körpers, da es instabil ist und das volle Körpergewicht trägt. Häufig sind auch muskuläre Disbilancen Ursache für Schmerzen an der vorderen Kniescheibe. Vor allem bei Personen mit sitzenden Berufen ist die ischiokrurale Muskulatur meist verkürzt und zieht die Gelenkkapsel nach hinten, wodurch sich der Anpreßdruck der Kniescheibe unphysiologisch erhöht. Sportverletzungen des Kniegelenkes entstehen vor allem beim Fußballspielen und beim Skilaufen durch plötzliche Drehungen und Belastungen des Unterschenkels. Affektionen des Knies haben meist örtliche Schmerzen zur Folge. Die Komplexität des Gelenks erschwert die genaue Diagnose. Deshalb kommt es häufig vor, daß Patienten nicht die richtige Anfangsbehandlung erhalten, was zu späteren degenerativen Erkrankungen führen kann.

Zur Versorgung von Knieverletzungen steht eine Vielfalt therapeutischer Hilfsmittel zur Ruhigstellung oder Bewegungseinschränkung sowie zur Stützung und Entlastung des Kniegelenkes zur Verfügung. Auch der Bereich der Aktiv-Bandagen ist mit einer Vielzahl von Produkten besetzt, wie sie bei keinem anderen Körperteil zu finden ist. So ist durch die dem Stand der Technik am nächsten kommende und die Ansprüche 1, 3 bis 5 betreffende DE-A- 34 16 231 eine Kniebandage zum Schlüpfen aus elastischem Material mit gegebenenfalls eingearbeiteten, längsverlaufenden Federn zur Verstärkung der Medial- und Lateralseite des Kniegelenkes, eine Aussparung für die Patella und eine diese Aussparung umgebende Polsterung bekannt, wobei die die Aussparung umgebende Polsterung derart ausgeführt ist, daß sich ein Anpreßdruck auf das Kniescheibenband ergibt, der das femoro-patellare Gleitlager entlastet. Die Polsterung umgibt dabei die Aussparung U-förmig, wobei die U-Form nach oben geöffnet ist. Die Polsterung stellt ein elastisches, in die Kniebandage eingearbeitetes U-Element dar, das die Aussparung ausschließlich seitlich und von unten umgibt. Dieses Element besteht aus Silikonmaterial oder Gummi. Des weiteren ist das U-Element als Wulst mit konvexer Seite zum Körper ausgebildet. Wesentlich ist jedoch bei dieser Kniebandage, daß das Hauptvolumen der Polsterung bzw. des Wulstes über dem Kniescheibenband angeordnet ist. Neben einem angenehmen Tragegefühl soll eine optimale Entlastung des Kniegelenkes bei gleichzeitiger Führung desselben erreicht werden, um hierdurch im Falle von Verletzungen, Reizungen oder anderen pathologischen Zuständen einen möglichst kurzen und schonenden Heilungsvorlauf zu erreichen. Allerdings erfolgt bei dieser Kniebandage keine Druckentlastung auf das Kniescheibenband, denn es soll ja gerade ein Anpreßdruck auf das Kniescheibenband erreicht werden. Die Kniegelenkbandage nach DE-U-90 04 974 besteht aus einem sich konisch nach unten verjüngenden Kniestrumpf aus einem elastischen Material, wie einem Gummigewebe. Der Kniestrumpf trägt auf seiner Vorderseite im Bereich der Kniescheibe zwei von oben sich nach unten erstreckende Halterungen, die die Kniescheibe seitlich auf ihrer Unterseite anliegend abstützen. Diese Kniegelenkbandage soll eine höhenmäßige Veränderung der Kniescheibe zu den Muskeln erlauben, jedoch die Kniescheibe gegenüber der Gleitfläche in angehobenem Zustand durch eine unterseitige Abstützung der Kniescheibe halten.

Die DE-A- 39 91 334 beschreibt eine elastische Kniegelenkbandage in Schlauchform mit einer elastischen, die Kniescheibe in einer Aussparung umfassenden Profileinlage. In die Profileinlage ist in festem Verbund mit dieser ein biegsames, nicht dehnbares Spannglied eingelagert, das die Bereiche der Profileinlage benachbart zu den Kniescheibenpolen wadenbeinseitig im Bogen um die Kniescheibe derart verbindet, daß bei Vergrößerung der Entfernung dieser Bereiche beim Beugen des Kniegelenkes der Abstand des Bogens von der Verbindungslinie der Kniescheibenpole verringert wird und der betreffende Rand der Aussparung der Profileinlage auf die benachbarte Seite der Kniescheibe diese medial verschiebend und zentrierend drückt. Mit dieser Kniegelenkbandage soll auf eine Kniescheibe, die aus ihrer Idealposition entweder krankhaft oder als sehr häufige Normvariante wadenbeinseitig verschoben ist, beim Beugen des Kniegelenkes dynamisch eingewirkt werden, um dabei die Kniescheibenlage zu korrigieren. Beim Beugen des Kniegelenkes wird jedoch die bei dieser Kniegelenkbandage verwendete Spange auseinandergezogen mit der Folge, daß sich die Spange aufrichtet und die gewünschte zentrierende Wirkung nicht voll erreicht wird.

Durch die dem Stand der Technik am nächsten kommende und den Anspruch 2 betreffende FR-A- 2 607 384 ist eine Bandage mit einer ringförmigen Pelotte mit einem stabförmigen Ansatz bekannt, der von zwei erhaben ausgebildeten Abschnitten gebildet wird, die über einen Steg miteinander verbunden sind. Dieser Steg weist gegenüber der Stärke der eigentlichen Ringpelotte eine geringere Stärke auf. Die Ringpelotte selbst ist als flacher Formkörper ausgebildet, an den sich der Steg mit den beiden sich in Steglängsrichtung erstreckenden erhaben ausgebildeten Abschnitten anschließt, wobei anstelle von wulstförmigen Abschnitten, die den Steg seitlich begrenzenden Ränder verstärkt mit einer Höhe, die der Materialstärke der Ringpelotte entspricht, sich zum Stegende hin verjüngen. Hinzu kommt, daß die erhaben ausgebildeten Abschnitte bzw. Längsränder der Ringpelotte parallel zueinander verlaufen. Eine wulstartige Ausbildung und ein bogenförmiger Verlauf der im Stegbereich sich gegenüberliegenden und den Steg seitlich begrenzenden Enden der Ringpelotte ist bei der Ringpelotte der bekannten Gelenkbandage nicht vorgesehen.

Diese Bandage ist mit einer Patella-Öffnung versehen, die durch die Ringpelotte abgepolstert ist. Die Ränder beiderseits des Kniescheibenbandes sind parallel ausgerichtet. Dies reicht aus, um eine lose Führung zu erzielen. Die Länge des Schlitzes oder Kanals ist mindestens dem Querschnitt der Ringpelotte entsprechend definiert. So wird die Sehne über eine ausreichende Länge geführt. Außerdem weist die Ringpelotte im Bereich des unteren Bandes immer die selbe Höhendifferenz zu den seitlichen Verstärkungen und dem dazwischenliegenden Steg auf.

Die EP-A-498 062 beschreibt ein aus textilen Fäden hergestelltes Flächengebilde zur Verwendung im Beugebereich von Gelenkbandagen, das sich durch eine zumindest auf einer Seite ausgebildete Querwellenstruktur auszeichnet, die durch eine eingearbeitete oder unter einer Deckstruktur liegende, elastische Fadenanordnung, die in vorbestimmten Abständen mit der Deckstruktur verbunden ist, elastisch vorgespannt und stabilisiert ist, um in Form eines Einsatzes im Beugebereich eines Gelenkes einer Faltenbildung während des Beugevorganges entgegenwirken zu können, um das Auftreten von Falten im innenliegenden Gelenkbeugebereich zu vermeiden.

Der Erfindung liegt die Aufgabe zugrunde, eine Bandage für das Kniegelenk, im nachfolgenden als Genu-Kniegelenkbandage, und eine darauf aufbauende, nachfolgend als Patella-Kniegelenkbandage bezeichnete Kniegelenkbandage für den Einsatz bei einer Lateralisationstendenz der Patella zu schaffen, mit der folgendes erreicht werden soll:
- Unterstützung der Physiotherapie. Wichtiger Bestandteil der krankengymnastischen Behandlung ist die Dehnung der ischiokruralen Muskeln.
- Entlastung des Kniegelenkes und der Patella.
- Herbeiführung einer raschen Abschwellung, Schmerzlinderung und Funktionsverbesserung.
- Ausübung einer intermittierenden Kompression und einer Massage auf die Gelenkweichteile.
- Vermeidung eines unerwünschten Druckes auf die Patella und den darunterliegenden Knorpelbelag.
- Verringerung des Anpreßdruckes auf die Patella.
- Erhaltung der physiologischen Gleitfähigkeit der Patella.
- Entlastung des Ansatzes der ischiokruralen Muskeln.
- Vermeidung eines unerwünschten Druckes auf die Nerven und Gefäße, die in der Kniekehle verlaufen.
- Vermeidung von negativen Auswirkungen auf die Muskelaktivität.
- Vermeidung einer Faltenbildung der Bandage in der Kniekehle.
- Keine Beeinträchtigung der Funktion der Quadrizeps-Sehne, die Patella in ihre richtige Position zu ziehen,
   wobei die Patella-Kniegelenkbandage darüber hinaus folgende Eigenschaften haben soll:
   - Seitliche Führung der Patella zur Verringerung der Lateralabweichung.

Gelöst wird diese Aufgabe bei einer Bandage für Kniegelenke durch die in den Ansprüchen 1,2,3,4 und 5 angegebenen Merkmale.

Eine Genu-Kniegelenkbandage gemäß der eingangs genannten Art ist nach Anspruch 1 in der Weise ausgebildet, daß das Gewebe oder Gestrick der Bandage einen rundumlaufenden Einsatz aus einem hochelastischen Wellengestrick aufweist, wobei die nach oben offene Pelotte mit einem bogenförmigen Verlauf der Außenränder der beiden Pelottenschenkel und mit einer außenseitig liegenden, ebenen Grundfläche sowie mit einem halbkreis- oder teilkreisförmigen Schenkelquerschnitt mittig in dem die beiden Pelottenschenkel verbindenden Steg eine innenseitig liegende Vertiefung zur Anpassung an den Schienbeinkopf und an den Verlauf des Kniescheibenbandes aufweist, wobei die Länge der Vertiefung der Breite des Pelottensteges entspricht.

Neben der U-förmigen Pelotte kann auch eine ringförmige Pelotte bei einer Kniegelenkbandage nach Anspruch 2 eingesetzt werden, die in der Weise ausgebildet ist, daß das Gewebe oder das Gestrick der Bandage einen rundumlaufenden Einsatz aus einem hochelastischen Wellenstrick aufweist, wobei die Bandage mit mindestens einem eingearbeiteten, längsverlaufenden Federstab vorsehen ist und die ringförmige oder ovale Pelotte einem halbkreis- oder teilkreisförmigen Schenkelquerschnitt und mittig in dem die beiden Pelottenschenkel verbindenden Steg eine innenseitig liegende Vertiefung aufweist, wobei die Länge der Vertiefung der Breite des Pelottensteges entspricht, wobei die die stegartige Vertiefung begrenzenden seitlichen Ränder geradlinig oder kreisbogenförmig verlaufend sind und wobei die im Bereich der seitlichen Ränder endenden Abschnitte der Pelottenschenkel zur Erzielung einer Friktion zu beiden Seiten des Sehnenansatzes eine wulstartige Ausgestaltung aufweisen.

Die Patella-Kniegelenkbandage ist entsprechend der Genu-Kniegelenkbandage ausgebildet, die nach Anspruch 3 derart ausgebildet ist, daß das Gewebe oder Gestrick der Bandage einen rundumlaufenden Einsatz aus einem hochelastischen Wellengestrick aufweist, wobei in dem vorderen Bandagenteil mindestens eine eine nach oben offene oder ringförmige die Pelotte stabilisierende, in Bandagenlängsrichtung verlaufende Pelottenstabilisierungsspange angeordnet ist und wobei die Pelottenstabilisierungsspange integrierter Bestandteil der Pelotte oder seitlich zu dieser liegend ist.

Die Kniegelenkbandage nach Anspruch 4 ist in der Weise ausgebildet, daß das Gewebe oder Gestrick der Bandage einen rundumlaufenden Einsatz aus einem hochelastischen Wellengestrick aufweist und daß in das rückwärtige Bandagenteil eine zweite oder eine zweite und eine dritte, den Ansatz der ischiokruralen Muskulatur beaufschlagende Pelotte aus einem weichen oder weich-elastischen Material eingesetzt ist.

Eine weitere Ausführungsform der Kniegelenkbandage ist nach Anspruch 5 derart ausgebildet, daß das Gewebe oder Gestrick der Bandage einen rundumlaufenden Einsatz aus einem hochelastischen Wellengestrick aufweist, wobei in dem vorderen Bandagenteil mindestens eine, eine nach oben offene oder ringförmige Pelotte stabilisierende, in Bandagenlängsrichtung verlaufende Pelottenstabilisierungsspange angeordnet ist, wobei die Pelottenstabilisierungsspange integrierter Bestandteil der ersten Pelotte oder seitlich zu dieser liegend ist. In das rückwärtige Bandagenteil sind eine zweite oder eine zweite und eine dritte, den Ansatz der ischiokruralen Muskulatur beaufschlagende Pelotte aus einem weichen oder weichelastischen Material eingesetzt sind.

Mit einer derart ausgebildeten Bandage für das Kniegelenk lassen sich folgende Indikationen am Knie wirkungsvoll behandeln.
- Reizzustände und Überlastungserscheinungen des Kniegelenks.
- Distorsionen und Kontusionen.
- Gelenkergüsse und Schwellungen bei Arthrose und Arthritis.
- Myotendopathien.
- Bänderschwäche.
- Patellaspitzensydrom.
- Luxationsneigung der Patella.
- Lateralisationstendenz der Patella.
- Chondropathia patellae.

Der Indikationsrahmen "schmerzhafte Reizzustände des Kniegelenks" ist sehr weit gefaßt und reicht von Weichteilschwellungen nach Sport- oder Arbeitsunfällen über chronische Beschwerden bis zu femoropatellaren Dysplasien mit Lateralisationstendenz. Letztere erfordern Konstruktionsmerkmale einer Kniebandage, die über die zur Therapie allgemeiner Kniebeschwerden nötigen Eigenschaften hinausgehen. Alle Wirkfaktoren in ein Produkt einzubauen, würde sich restriktiv auf den Indikationsbereich auswirken: Die Bandage wird zur Spezialbandage und würde z.B. bei unspezifischen Knieschmerzen eine Überversorgung darstellen. Deshalb ist es vorteilhaft, wenn zwei Bandagen eingesetzt werden, nämlich die Genu-Kniegelenkbandage als allgemeines Basis-Produkt für Überlastungserscheinungen, femoropatellare Schmerzsyndrome und für das Patellaspitzensyndrom, während die Patella-Kniegelenkbandage das auf dieser Genu-Kniegelenkbandage aufbauende Produkt darstellt und bei Lateralisationstendenz und chondropathischen Beschwerden eingesetzt wird. Beide Bandagen, die Genu-Kniegelenkbandage und die Patella-Kniegelenkbandage bauen aufeinander auf.

Deshalb sind die Wirkungsweisen und Vorteile für beide Bandagen weitgehend identisch, wobei für die Patella-Kniegelenkbandage außerdem noch weitere Vorteile hinzu kommen.

Mit der Genu- und Patella-Kniegelenkbandage wird die Physiotherapie unterstützt und das Kniegelenk und die Patella entlastet. Eine rasche Abschwellung, Schmerzlinderung und Funktionsverbesserung wird herbeigeführt. Darüber hinaus wird eine intermittierende Kompression auf die Gelenkweichteile und eine tiefenwirksame Massage ausgeübt. Ein unerwünschter Druck auf die Patella und den darunterliegenden Knorpelbelag wird vermieden. Der Anpreßdruck der Patella wird verringert, wobei deren physiologische Gleitfähigkeit erhalten wird. Der Ansatz der ischiokruralen Muskeln wird entlastet. Ein unerwünschter Druck auf die Nerven und Gefäße, die in der Kniekehle verlaufen, wird vermieden. Es ergeben sich keine negativen Auswirkungen auf die Muskelaktivität. Die Bandage schnürt nicht und ruft keine zirkulatorischen Schwierigkeiten hervor. Eine Faltenbildung der Bandage in der Kniekehle bei einer Kniebeugung wird minimiert. Die Bandage rutscht nicht und auch bei Bewegung wird ein kontinuierlich guter Sitz beibehalten. Mit der Bandage wird ein hoher Tragekomfort erreicht, da sie nicht scheuert und hautfreundlich ist. Außerdem hat die Kniegelenkbandage den Vorteil, daß die Funktion der Quadrizeps-Sehne, die Patella in ihre richtige Position zu ziehen, nicht beeinträchtigt wird. Außerdem erfolgt bei der Patella-Kniegelenkbandage eine seitliche Führung der Patella, wobei die Lateralabweichung verringert wird.

Die Genu-Kniegelenkbandage ist eine dreidimensional anatomisch formgestrickte Zweizug-Bandage, die vom Unterschenkel bis zum Oberschenkel reicht und eine funktionale leichte Beugestellung von etwa 10° besitzt und mit Druckminderungsrändern ausgestattet ist. Die Bandage selbst besteht aus zwei Teilen, wobei das vordere Bandagenteil 2/3 des Beinumfanges umspannt. Über dem Kniegelenk befindet sich ein um das ganze Bein reichender Einsatz aus hochelastischem Wellengestrick. Das rückwärtige Bandagenteil wird mit vorgespannter Welle des Wellengestricks eingenäht.

Über den seitlichen Nähten in Taschen eingearbeitete Federstäbe halten die Bandage gespannt. Das Wellengestrick ist über der Patella geschlossen. Im Bereich über der Kniescheibe befindet sich eine bevorzugterweise hufeisenförmig ausgebildete erste Pelotte. Die nach oben geöffnete Pelotte läßt die Quadrizepssehne frei. Im unteren Teil ist das Profil der Pelotte dem Schienbeinkopf und dem Verlauf des Ligamentum patellae angepaßt. Diese im vorderen Bandagenteil angeordnete Pelotte enthält bevorzugterweise einen knopfförmigen Friktionskern aus einem Material, welches gegenüber dem Material der Pelotte einen höheren Härtegrad aufweist. Dieser Friktionskern ist infrapatellar angeordnet. In das rückwärtige Bandagenteil ist eine Pelotte oder sind zwei Pelotten eingenäht, die ellipsenförmig ausgebildet sind oder die andere geometrische Formen aufweisen und die den Ansatz der ischiokruralen Muskulatur beaufschlagen. Diese beiden Pelotten sind etwa 1 cm oberhalb des Kniegelenkspalts unmittelbar an den seitlichen Nähten plaziert, vermittels der das vordere Bandagenteil mit dem rückwärtigen Bandagenteil verbunden ist.

Auch die Patella-Kniegelenkbandage ist eine dreidimensional anatomisch formgestrickte Zweizug-Bandage für das Kniegelenk. Die Bandage reicht vom Unterschenkel bis zum Oberschenkel und hat eine funktionelle leichte Beugestellung von etwa 10° und ist mit Druckminderungsrändern ausgestattet. Die Bandage besteht ebenfalls aus zwei Teilen, wobei das Vorderteil 2/3 des Beinumfangs umspannt. Über dem Kniegelenk befindet sich ein um das ganze Bein reichender Einsatz aus hochelastischem Wellengestrick. Das rückwärtige Bandagenteil wird mit vorgespannter Welle eingenäht. Über den seitlichen Nähten in Taschen eingearbeitete Federstäbe halten die Bandage gespannt. Das Gestrick ist über der Patella geschlossen. Im Bereich über der Kniescheibe befindet sich eine bevorzugterweise hufeisenförmige erste Pelotte. Die nach oben geöffnete Pelotte läßt die Quadrizeps-Sehne frei. Im unteren Teil ist das Profil der Pelotte dem Schienbeinkopf und dem Verlauf des Ligamentum patellae angepaßt. Die bei der Patella-Bandage verwendete Pelotte kann einen knopfförmigen Friktionskern enthalten, der aus einem Material besteht, dessen Härtegrad höher ist als der Härtegrad des Materials, aus dem die Pelotte hergestellt ist. Dieser Friktionskern ist dann infrapatellar angeordnet. Außerdem kann die Pelotte der Patella-Kniegelenkbandage eine Komponente mit unterschiedlichen Dehnungseigenschaften aufweisen. Die erste Pelotte ist seitlich mit einem fächerartigen Streifen bzw. mit einer Spange verbunden, die bevorzugterweise aus einem federnd-elastischen Material besteht, jedoch keine Längselastizität aufweist. Wenn es bei der Bewegung zwischen Streckung und Beugung zu einer Verlängerung der elastischen Elemente in der Bandage kommt, zieht sich dieser Streifen bzw. die Spange gerade und übt eine zentrierende Kraft auf die Pelotte aus. In das rückwärtige Bandagenteil der Patella-Kniegelenkbandage können zwei ellipsenförmige Pelotten eingenäht sein, die den Ansatz der ischiokruralen Muskulatur beaufschlagen. Diese beiden Pelotten sind etwa 1 cm oberhalb der Kniegelenkspalts unmittelbar an den seitlichen Nähten plaziert.

Vorteilhafte Ausgestaltungen der Erfindung sind in den Unteransprüchen gekennzeichnet.

Besonders vorteilhaft ist die Ausgestaltung des Wellengestrickes, aus dem der Einsatz besteht, der im Beugebereich der Bandage angeordnet ist. Dieses Wellengestrick besitzt zumindest auf einer Seite ein Relief in Form einer Wellenstruktur. Diese Wellenstruktur wird durch eine darunterliegende Fadenanordnung aus höherelastischem Garn bzw. Faden elastisch vorgespannt und stabilisiert, wodurch die Deckstruktur im entspannten Zustand des Wellengestricks wellenartig, vorzugsweise halbwellenartig, ausbaucht. Wenn dementsprechend diese Flächenstruktur bei einer Bandage eingesetzt und senkrecht zur Ausrichtung der Querwellen beansprucht wird, was beispielsweise beim Beugen des bandagierten Gelenkes der Fall ist, so werden zunächst lediglich die Querwellen flacher bzw. glatt gezogen , ohne daß eine Streckung bzw. Reckung der Deckstruktur erfolgt. Überdehnungen der Deckstruktur können auf diese Art und Weise zuverlässig ausgeschlossen werden, wodurch gleichzeitig das Auftreten von Falten, und zwar selbst nach längerem Gebrauch der Bandage, im innenliegenden Gelenkbeugebereich vermieden wird. Das Wellengestrick eignet sich deshalb insbesondere zur Verwendung in Bandagen, die für die Gelenke mit großem Bewegungsfreiraum bzw. Beugewinkel eingesetzt werden, wie das beispielsweise bei Kniegelenkbandagen der Fall ist. Besonders vorteilhaft ist dabei die Ausgestaltung, nach der das Wellengestrick eine zumindest auf einer Seite ausgebildete, im wesentlichen senkrecht zur Hauptdehnungsrichtung ausgerichtete Wellenstruktur einer Deckstruktur aufweist, wobei diese Wellenstruktur durch eine eingearbeitete oder unter dieser Deckstruktur liegende elastische Fadenanordnung elastisch vorgespannt und stabilisiert ist, die in vorbestimmten Abständen mit der Deckstruktur verbunden ist und derart geformt ist, daß die Anzahl der Querwellen dort am größtem ist, wo aufgrund der Gelenkbeugung der größte Dehnungsweg auftritt.

Ein derartiges Wellengestrick erlaubt somit trotz verhältnismäßig hohen Dehnungsvermögens die Verwendung von verhältnismäßig unelastischem Garn für die Deckstruktur, wodurch die Voraussetzung für eine wirtschaftliche Herstellung des Wellengestricks geschaffen wird. Des weiteren besteht bei dem Wellengestrick keine Gefahr von Überdehnungen des eingesetzten Garns, da eine große Grund-Elastizität vorhanden ist.

Ausführungsbeispiele der Erfindung werden nachstehend anhand der Zeichnung näher erläutert. Es zeigen
Fig. 1 in einer Ansicht von vorn eine Genu-Kniegelenkbandage mit einer U-förmigen Pelotte mit in deren rückwärtigen Bandagenteil angeordneten Federstäben und zwei ellipsenförmigen Pelotten,
Fig. 2 eine Rückansicht der Genu-Kniegelenkbandage,
Fig. 3 in einer Ansicht von vorn eine Patella-Kniegelenkbandage mit einer U-förmigen Pelotte und mit einer in einem Abstand von dieser seitlich angeordneten Pelottenzentrierungsspange und mit im rückwärtigen Bandagenteil angeordneten Federstäben und zwei ellipsenförmigen Pelotten,
Fig. 4 eine Rückansicht der Patella-Kniegelenkbandage,
Fig. 5 in einer Ansicht von vorn eine weitere Ausführungsform der Patella-Kniegelenkbandage mit einer U-förmigen Pelotte und mit einer seitlich von dieser angeordneten Pelottenzentrierungsspange,
Fig. 6 in einer Ansicht von vorn eine weitere Ausführungsform der Patella-Kniegelenkbandage mit einer U-förmigen Pelotte und mit einer in deren einen Pelottenschenkel integrierten Pelottenzentrierungsspange,
Fig. 7 in einer Ansicht von vorn die U-förmige Pelotte mit einer in deren einen Pelottenschenkel integrierten Pelottenzentrierungsspange,
Fig. 8 in einer Ansicht von vorn die U-förmige Pelotte mit einer an deren einen Pelottenschenkel angeformten Pelottenzentrierungsspange,
Fig. 9 in einer Ansicht von vorn eine Genu-Kniegelenkbandage mit einer U-förmigen, einen Friktionskern aufweisenden Pelotte,
Fig. 10 in einer Ansicht von vorn eine einen Friktionskern aufweisende U-förmige Pelotte,
Fig. 11 in einer Ansicht von vorn die U-förmige Pelotte,
Fig. 12 einen senkrechten Schnitt gemäß Linie XII-XII in Fig. 11,
Fig. 13 einen senkrechten Schnitt gemäß Linie XIII-XIII in Fig. 11,
Fig. 14 in einer Ansicht von vorn eine ellipsenförmige Pelotte,
Fig. 15 einen senkrechten Schnitt gemäß Linie XV-XV in Fig. 14,
Fig. 16 in einer Ansicht von vorn zwei über einen Steg miteinander verbundene ellipsenförmige Pelotten,
Fig. 17 einen senkrechten Schnitt gemäß Linie XVII-XVII in Fig. 16,
Fig. 18 in einer Ansicht von vorn eine U-förmige Pelotte mit seitlichen, über Stege mit der Pelotte verbundenen ellipsenförmigen Pelotten,
Fig. 19 einen senkrechten Schnitt gemäß Linie XIX-XIX in Fig. 18,
Fig. 20 in einer Ansicht von vorn eine Patella-Kniegelenkbandage mit einer U-förmigen Pelotte und mit zwei seitlich zu dieser angeordneten Pelottenzentrierungsspangen,
Fig. 21 in einer schematischen Ansicht von vorn einen in der Bandage rundum angeordneten Einsatz aus einem hochelastischen Wellengestrick,
Fig. 22 in einer Rückansicht den Einsatz aus dem hochelastischen Wellengestrick,
Fig. 23 in einer schematischen Darstellung die Ausbildung des hochelastischen Wellengestricks in dem vorderen Bandagenteil und in dem rückwärtigen Bandagenteil der Kniegelenkbandage,
Fig. 24 einen vergrößerten Schnitt durch den Einsatz aus dem Wellengestrick gemäß Linie XXIV-XXIV in Fig. 23,
Fig. 25 in einer schematisch vereinfachten Darstellung das Prinzip der Verformung eines aus dem Wellengestrick bestehenden Flächengebildes,
Fig. 26 Darstellungen von Strickreihen zur Verdeutlichung einer ersten Ausführungsform eines Verfahrens zur Herstellung des Wellengestricks,
Fig. 27 in einer der Fig. 26 ähnlichen Darstellung ein weiteres Strickmuster für das Wellengestrick,
Fig. 28 in einer Seitenansicht einen in der U-förmigen Pelotte angeordneten Friktionskern,
Fig. 29 eine Ansicht auf das menschliche Kniegelenk,
Fig. 30 in einem Querschnitt die rechte Tibia.
Fig. 31 in einer Ansicht von oben eine ringförmige Pelotte und
Fig. 32 einen senkrechten Schnitt gemäß Linie XXXII-XXXII in Fig. 31.

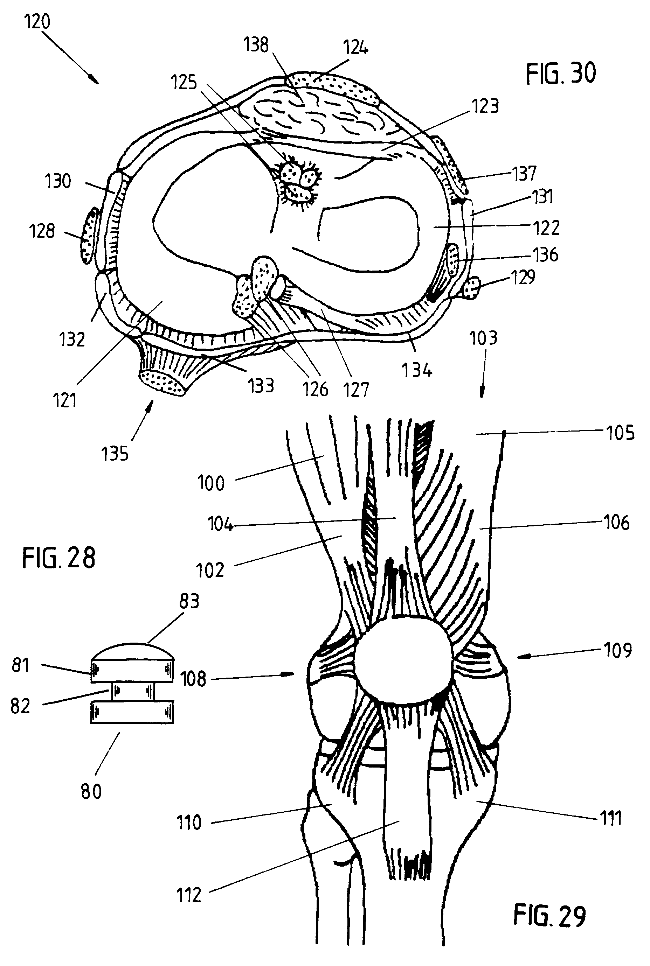

Das in der Mitte der Bewegungskette "untere Extremität" gelegene,in Fig. 29 wiedergegebene Kniegelenk ist das größte Gelenk des menschlichen Körpers. Der obere Gelenkkörper ist eine in der Mitte eingekerbte Rolle. Ihr steht eine nur vom Schienbein gebildete, sehr flache Gelenkfläche gegenüber. Die Menisken, zwei auf der Schienbeingelenkfläche liegende Knorpelplatten, stellen die Pfannen dar, in denen sich die Rolle des oberen Gelenkörpers scharnierartig bewegen kann. Eine knöcherne Führung des Kniegelenkes ist nicht vorhanden. Diese Funktion wird von anderen Strukturen übernommen. Die kräftigen Seitenbänder dienen als Führungsschienen für die Scharnierbewegung. Das Durchrutschen der gelenkbildenden Knochen nach hinten oder vorn verhindern die kurzen, im inneren des Gelenkes liegenden Kreuzbänder. Vor dem Gelenk liegt, in eine Sehne eingebettet, die Kniescheibe. Sie ist der größte freie Knochen und dient der Verbesserung der Zugwirkung des Unterschenkelstreckers. Die Patella-Sehne ist sehr empfindlich.

Bei dem in Fig. 29 dargestellten Kniegelenk ist mit 100 der Musculus vastus lateralis, mit 102 die Tendo musculi vasti lateralis, mit 103 der Musculus vastus intermedius, mit 104 der Musculus recto femoris, mit 105 der Musculus vastus medialis, mit 106 der Musculus vastus medialis obliquus, mit 107 die Patella, mit 108 das Ligamentum patellofemorale laterale, mit 109 das Ligamentum patellofemorale mediale, mit 110 das Ligamentum patellotibiale laterale, mit 111 das Ligamentum patellotibiale mediale und mit 112 das Ligamentum patellae bezeichnet.

Die Stabilisatoren des Kniegelenks werden in vier Funktionseinheiten dabei eingeteilt: Die Stabilisatoren des medialen und des lateralen Komplexes sowie die dorsalen und ventralen Strukturen, wobei zwischen statischen und dynamischen Stabilisatoren unterschieden wird.

Fig. 30 zeigt die rechte Tibia 120 in einem Querschnitt. Hier sind mit 121 der Innenmeniskus, mit 122 der Außenmeniskus, mit 123 das Ligamentum genus transversum, mit 124 das Ligamentum patellae, mit 125 das Ligamentum cruciatum anterius, mit 126 das Ligamentum cruciatum posterius, mit 127 das Ligamentum meniscofemorale posterius, mit 128 das Ligamentum collaterale mediale, mit 129 das Ligamentum collaterale laterale, mit 130 das mediale Kapselband, mit 131 das laterale Kapselband, mit 132 das Ligamentum obliquum posterius, mit 133 das Ligamentum popliteum obliquum, mit 124 das Ligamentum popliteum arcuatum, mit 135 der Musculus semimembranosus, mit 136 der Musculus popliteus, mit 137 der Tractus iliotibialis und mit 138 der Hoffasche Fettkörper bezeichnet.

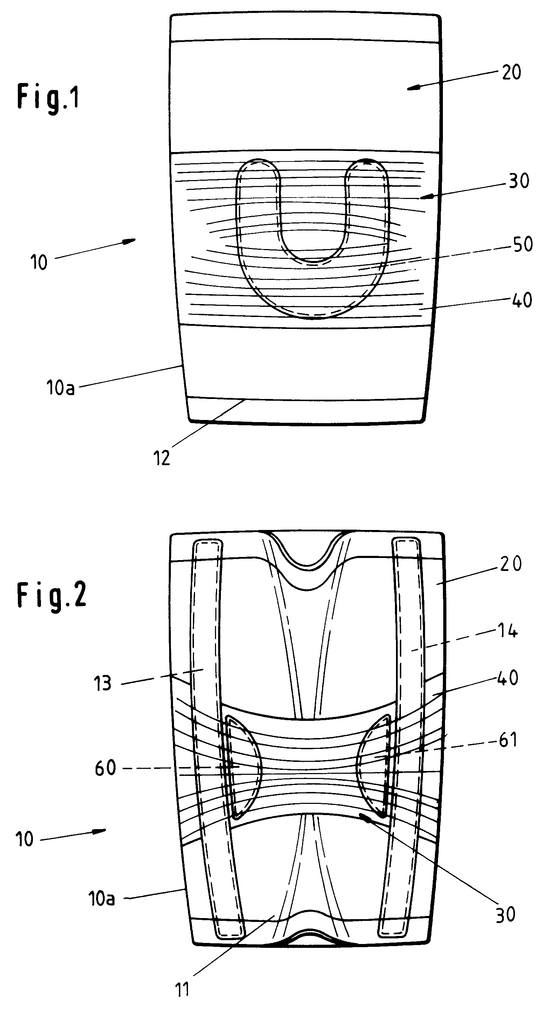

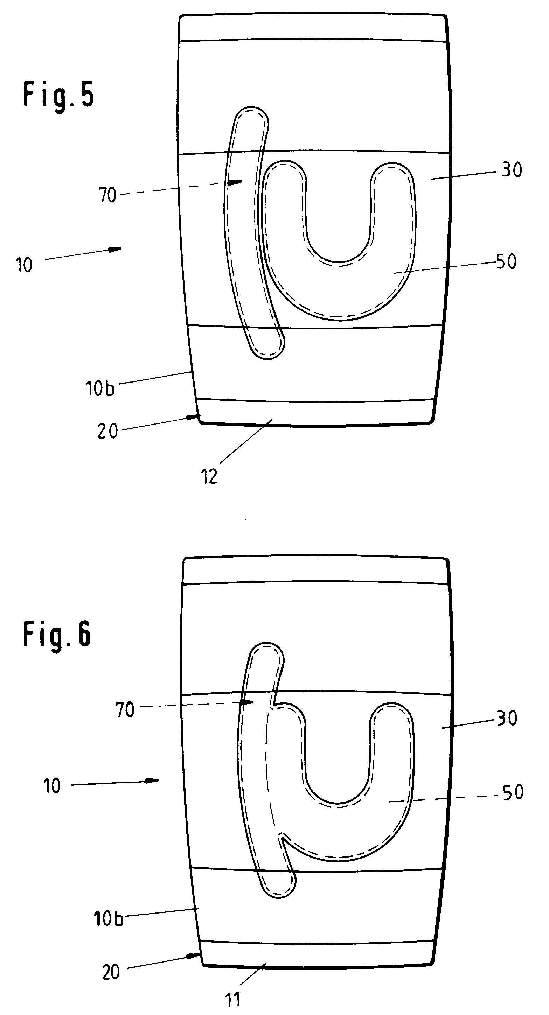

Die in der Zeichnung dargestellte Bandage 10 für ein Kniegelenk besteht aus einem anatomisch geformten Schlauchkörper 20 aus einem Gewebe oder Gestrick mit einem rundumlaufenden Einsatz 30 aus einem hochelastischen Wellengestrick 40. Über den das rückwärtige Bandagenteil 11 mit dem vorderen Bandagenteil 12 verbindenden Nähten sind Federstäbe 13,14 in das Schlauchkörpermaterial eingearbeitet, die entsprechend der anatomischen Form des Schlauchkörpers 20 bogenförmig verlaufend ausgebildet sind, wie dies in den Fig. 2 und 4 dargestellt ist. Diese Federstäbe 13,14 sind gerade verlaufend ausgebildet; sie werden erst hinterher den bogenförmigen Verlauf erhalten, wenn die Bandage ausgebildet wird.

In dem vorderen Bandangenteil 12 der Bandage 10 ist eine erste, nach oben offene und die Quatrizeps-Sehne freilassende Pelotte 50 aus einem weichen oder weich-elastischen Material angeordnet, wobei diese Pelotte 50 bei angelegter Bandage im Bereich über der Kniescheibe sitzt. Diese Pelotte 50 ist U-förmig ausgebildet und wird gebildet von den Schenkeln 51,52 und dem die Schenkel verbindenden Steg 53, wobei die Pelotte 50 auch V-förmig ausgebildet sein kann.

In das rückwärtige Bandagenteil 11 der Bandage 10 ist eine zweite oder eine zweite und eine dritte, den Ansatz der ischiokruralen Muskulatur beaufschlagende, ellipsenförmige Pelotte 60, 60' eingesetzt, die ebenfalls aus einem weichen oder weich-elastischen Material besteht.

Fig. 1 und 2 zeigen eine Bandage 10, die als Genu-Kniegelenkbandage 10a ausgebildet ist.

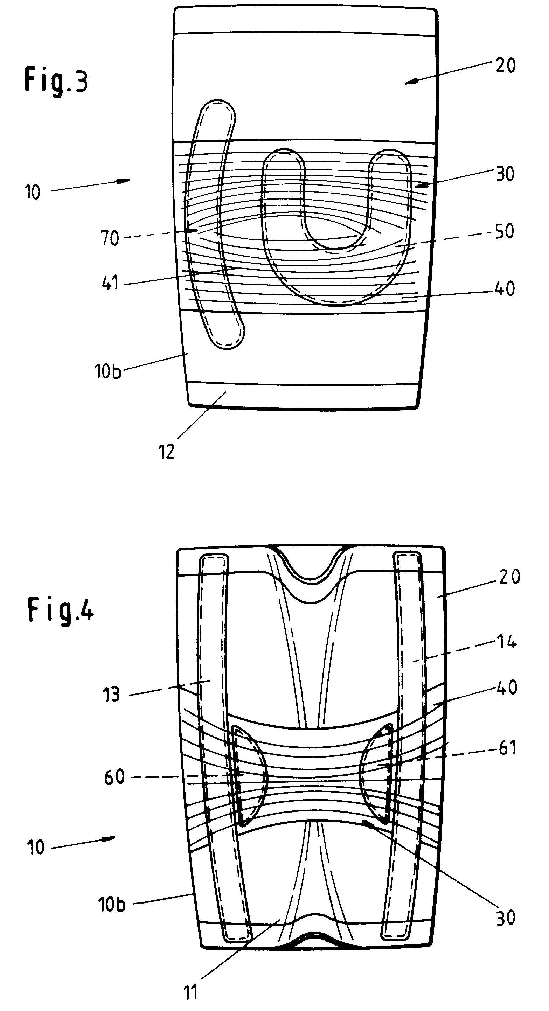

Als weitere Ausführungsform der Bandage 10 ist nach Fig.3 und 4 eine auf der Genu-Kniegelenkbandage aufbauende Patella-Kniegelenkbandage 10b vorgesehen, die sich von der Genu-Kniegelenkbandage 10a nur darin unterscheidet, daß zusätzlich in dem vorderen Bandagenteil 12 mindestens eine, die Pelotte 50 stabilisierende, in Bandagenlängsrichtung verlaufende elastische Spange 70 angeordnet ist, wobei diese Spange 70 integrierter Bestandteil der Pelotte 50 oder seitlich zu dieser liegend ist. Diese Spange 70 weist bevorzugterweise keine Längselastizität auf.

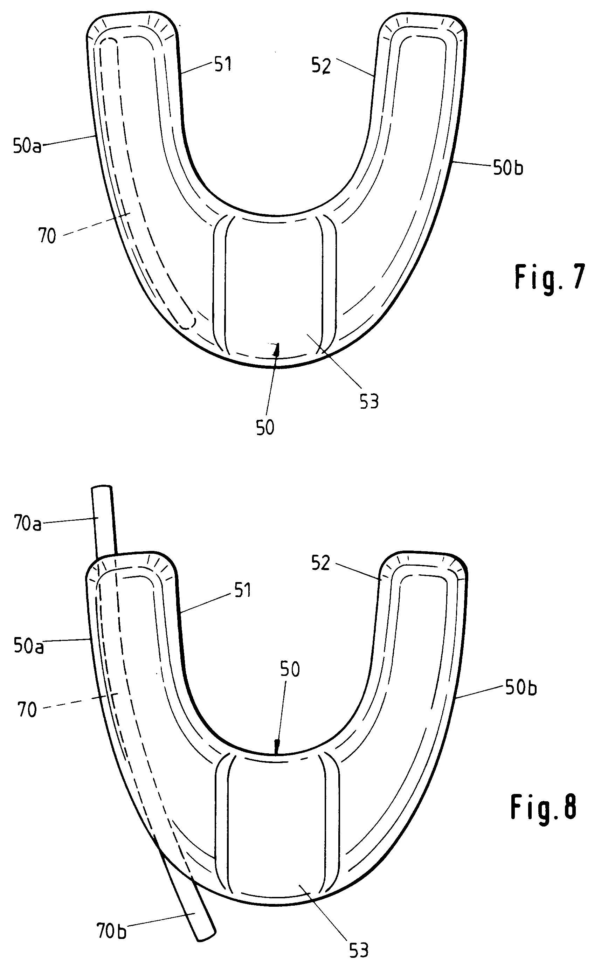

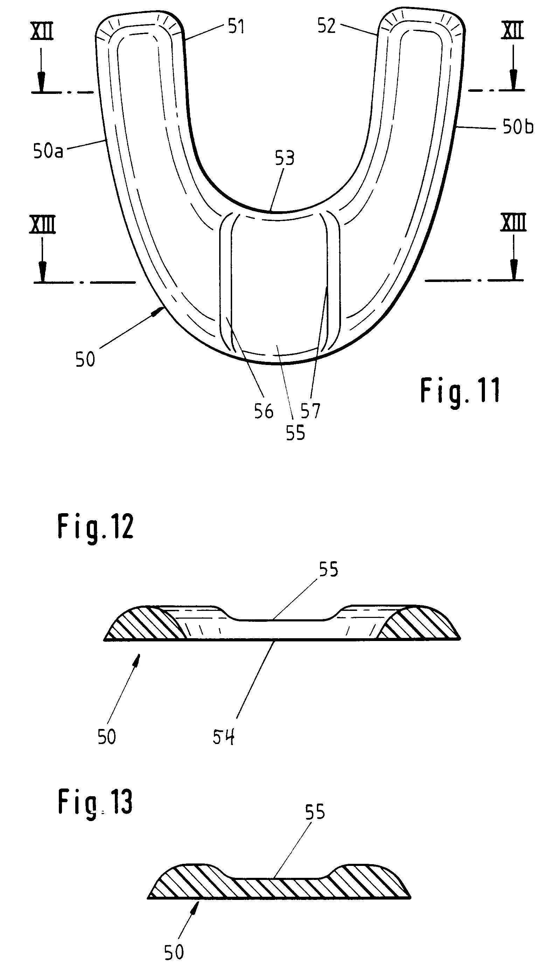

Die Pelotte 50 in dem vorderen Bandagenteil 12 weist nach einer ersten Ausführungsform eine hufeisenförmige Ausgestaltung mit einem bogenförmigen Verlauf der Außenränder 50a,50b der beiden Pelottenschenkel 51,52 auf (Fig.7,10 und 11). Die Pelotte 50 weist eine außenseitig liegende, ebene Grundfläche 54 auf; der Querschnitt der Pelottenschenkel 51,52 und der Querschnitt in einem Teilbereich des Pelottensteges 53 entspricht einem Halbkreis oder einem Teilkreis (Fig.12 und 13). In dem die Pelottenschenkel 51,52 miteinander verbindenden Steg 53 ist mittig eine innenseitig liegende Vertiefung 55 zur Anpassung an den Schienbeinkopf und an den Verlauf des Ligamentum patellae versehen. Die etwa rechteckförmige bzw. quadratische Vertiefung 55 weist in Bandagenlängsrichtung verlaufende seitliche Führungen 56,57 auf. Vermittels dieser Vertiefung 55 in der Pelotte 50 wird ein erhöhter Andruck auf das Kniescheibenband vermieden, wobei diese Profilierung der Pelotte 50 gleichzeitig eine Oberflächenmassage bewirkt. Die seitlichen Führungen 56,57 dienen zur Führung des Kniescheibenbandes. Wesentlich ist jedoch, daß diese Vertiefung 55 in der Pelotte 50 nicht nur zu einer Druckminderung führt, sondern auch der Kontur der Sehne angepaßt ist.

Die Pelotte 50 besteht aus Filz, Moosgummi, Neopren, Gummi, aus einem viskoelastischen Silikonkautschuk oder einem elastischen, kompressiblen, durch Druck verformbaren Silikonkautschuk oder einem Material mit gleichen Elastizitätseigenschaften, wie Naturkautschuk, Silikonschaum od.dgl..

Die Pelotte 50 kann auch beutelförmig ausgebildet sein und eine Füllung aus einem gasförmigen oder flüssigen Medium aufweisen.

Die Pelottenstabilisierungsspange 70 ist benachbart zu einem der beiden Schenkel 51,52 der Pelotte 50 liegend. Die Spange 70 entspricht in ihrer Formgebung der Form des äußeren Schenkelrandes 50a bzw. 50b der Pelotte 50 (Fig.3). Die Spange 70 kann in einem Abstand von dem Schenkel 51 der Pelotte 50 angeordnet sein. Zwischen dem Schenkel 51 der Pelotte 50 und der Spange 70 ist dann ein Gewebe- oder Gestrickabschnitt 41 angeordnet (Fig.3). Es besteht jedoch auch die Möglichkeit, die Spange 70 abstandslos neben dem Schenkel 51 der Pelotte 50 anzuordnen (Fig.5). Bei einer weiteren Ausführungsform ist die Spange 70 an dem Schenkel 51 der Pelotte 50 angeformt und somit Bestandteil der Pelotte 50 (Fig.6). Die Spange 70 und die Pelotte 50 sind dann einstückig ausgebildet, wobei die Spange 70 eine ihrer Wirkung entsprechende Formelastizität aufweist.

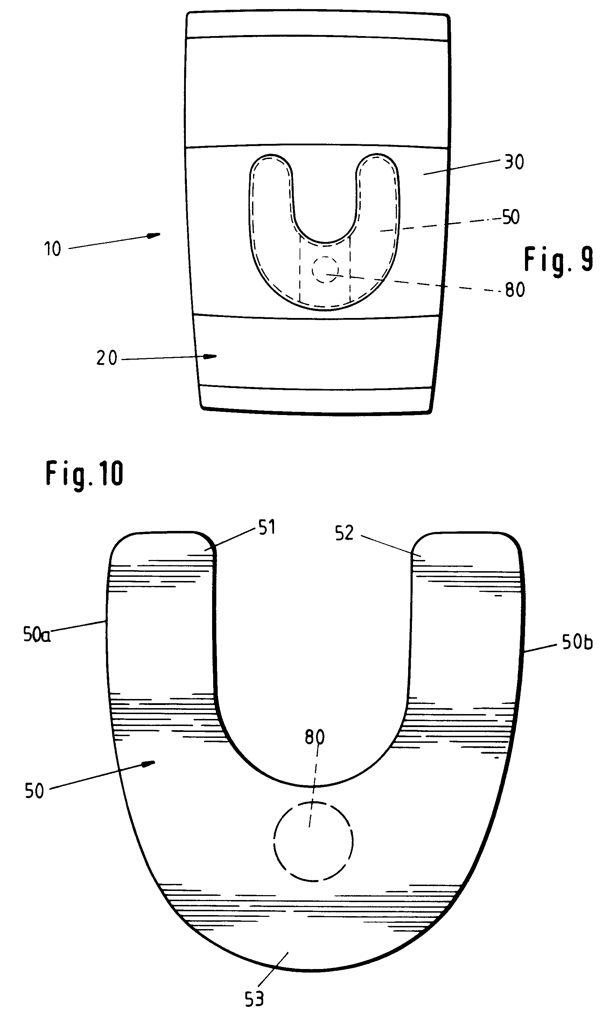

Die Spange 70 ist nach Fig.7 durch den Schenkel 51 der Pelotte 50 hindurchgeführt und in das Pelottenmaterial eingebettet. Bei der in Fig. 8 gezeigten Ausführungsform ist die Spange 70 in den Schenkel 51 der Pelotte 50 eingebettet, und zwar derart, daß die Spange 70 beidendseitig aus dem Pelottenschenkel 51 mit je einem Abschnitt 70a,70b herausragt.

Die Pelottenstabilisierungsspange 70 besteht bevorzugterweise aus einem federnd-elastischen Material, um sich allen Bewegungsabläufen anpassen zu können. Bevorzugterweise besteht die Spange 70 aus einem Kunststoff mit ausreichender Flexibilität und einem Härtegrad, der höher ist als der Härtegrad des Materials, aus dem die Pelotte 50 besteht. In Längsrichtung ist die Spange 70 unelastisch; sie kann jedoch auch in Längsrichtung elastisch sein.

In die Pelotte 50 ist ein knopfförmiger Friktionskern 80 eingearbeitet, der aus einem Material besteht, das gegenüber dem Härtegrad des Pelottenmaterials einen höheren Härtegrad aufweist. Dieser Friktionskern 80 ist in dem die beiden Schenkel 51,52 verbindenden Steg 53 der Pelotte 50, und zwar mittig , angeordnet (Fig. 9 und 10). Die Differenz zwischen der Härte der Pelotte 50 und der Härte des Friktionskerns 80 beträgt mindestens 10 Shore, bevorzugterweise 20 Shore A. Das Material der Pelotte 50 kann auch eine unterhalb 50 Shore A liegende Härte und das Material des Friktionskerns 80 eine oberhalb 50 Shore A liegende Härte aufweisen. Wesentlich ist, daß der Friktionskern 80 aus einem Material besteht, das gegenüber dem Material der Pelotte 50 etwas härter ist.

Während die Pelotte 50 aus einem weichen oder weich-elastischen Material, bevorzugterweise aus einem viskoelastischen Silikonkautschuk oder einem elastischen,kompressiblen,durch Druck verformbaren Silikonkautschuk, z.B. mit einer Härte von 40 Shore A, einem Silikonkautschuk mit einer Härte von 9 bis 13 Shore A oder einem kompressiblen, durch Druck verformbaren und ohne Sprungelastizität in seine Form sich rückbildenden Silikonkautschuk vom Typ eines nach dem Verfahren der Polyaddition vulkanisierenden Kaltkautschuk, der neben einer hohen Flexibilität eine unter 4 Shore A liegende Härte aufweist, wobei jedoch auch Silikonkautschuke eingesetzt werden können, deren Härte oberhalb von 4 Shore A liegt, besteht, besteht der Friktionskern 80 aus einem harten oder inkompressiblen Kunststoff. Derartige, für die Herstellung der Pelotte 50 verwendete viskoelastische Silikonkautschuke oder Materialien mit gleichen Elastizitätseigenschaften besitzen die Eigenschaft, bei angelegter Bandage mit einer derartigen Pelotte 50 aufgrund der gleitenden Bewegung, ausgelöst durch Masseverschiebung, bei Druckanwendung oder bei Bewegungsabläufen im Auflagebereich massierend zu wirken. Für die Herstellung der Pelotte 50 sollte immer ein Material gewählt werden, welches viskoelastisch ist und aufgrund seiner elastischen Eigenschaften eine Massage bewirkt.

Der Friktionskern 80 besteht gegenüber der Pelotte 50 aus einem harten oder inkompressiblen Kunststoff, mit z.B. einer über 50 Shore A liegenden Härte und weist somit gegenüber der Pelotte 50 eine weitaus größere Härte auf, damit bei einer Bewegung eine gezielte Friktionsmassage auf spezielle Schmerzpunkte des Gelenkes erreicht wird. Als Material für den Friktionskern 80 kommt natürlicher oder künstlicher Kautschuk oder Hartgummi infrage. So kann u.a. ein Chloropren-Polymerisat (Handelsname NEOPREN) mit einer Härte von 50 Shore A , ein gummielastisches, vernetztes Polyurethan (Handelsname VULKOLLAN) mit einer Härte von 65 bis 90 Shore A, ein Silikonkautschuk mit einer Härte von 60 Shore A, ein Ethylen-Propylen-Dien-Kautschuk (EPDM) mit einer Härte von 80 Shore A oder ein Copolymerisat mit Acrylnitril (Handelsname PERBUNAN) mit einer Härte von 70 Shore A, ferner ein Polyamid verwendet werden. Jedoch auch andere Kunststoffe oder Naturstoffabkömmlinge können zur Herstellung des Friktionskerns 80 herangezogen werden. Wesentlich ist, daß der Friktionskern 80 eine ausreichende Härte aufweist, um die gezielte Friktionsmassage auf spezielle Schmerzpunkte ausüben zu können. Der Friktionskern 80 kann auch aus Metall oder Holz bestehen.

Der Friktionskern 80 kann in der Pelotte 50 auswechselbar angeordnet sein. Hierzu ist die Pelotte 50 mit einer in der Zeichnung nicht dargestellten Ausnehmung von etwa der Größe des Friktionskerns 80 versehen, in die der Friktionskern 80 vermittels eines leichten Druckes eingedrückt wird. Die die Ausnehmung in der Pelotte 50 begrenzte Innenwandfläche weist bevorzugterweise eine Profilgebung auf, die einen Eingriff in das Profil der umlaufenden Wandfläche des Friktionskerns 80 ermöglicht und da das Pelottenmaterial elastisch ist, der Friktionskern 80 jedoch gegenüber dem Pelottenmaterial eine größere Härte aufweist, läßt sich der Friktionskern 80 in die Ausnehmung pressen, wobei während des Preßvorganges das Profil der Innenwandfläche so zusammengepreßt wird, daß der Friktionskern 80 gänzlich in die Ausnehmung gleiten kann und aufgrund des Rückstellvermögens des Materials der Pelotte 50 wird das Pelottenmaterial in das Randprofil des Friktionskerns 80 gepreßt, so daß dieser fest in der Pelotte 50 gehalten wird. Durch entsprechende Verformung der Pelotte 50 durch eine starke äußere Druckanwendung kann der Friktionskern 80 aus der Pelotte 50 herausgedrückt werden. Dadurch ist die Möglichkeit gegeben, Friktionskerne unterschiedlicher Härte verwenden zu können. In dem Fall, in dem Pelotten 50 mit auswechselbaren Friktionskernen 80 verwendet werden, ist die Pelotte 80 an dem Schlauchkörper 20 der Bandage 10 derart befestigt, daß ein Abnehmen der Pelotte 50 möglich ist.

Der Friktionskern 80 entsprechend Fig. 28 mit einem kreisförmigen, quadratischen, rechteckförmigen oder eine andere geometrische Form aufweisenden Querschnitt weist im Bereich seiner umlaufenden Wandfläche 81 eine Einziehung in Form einer Nut 82, rillenförmige Ausnehmungen, Hinterschneidungen, Verzahnungen od.dgl. auf, die zur Aufnahme des Pelottenmaterials dient, damit der Friktionskern 80 in dem Formkörper der Pelotte 50 in seiner Lage fixiert ist. Bevorzugterweise ist die Anordnung des Friktionskerns 80 in der Pelotte 50 so getroffen, daß dieser dicht unterhalb der Oberfläche der Pelotte 50 zu liegen kommt, um so einen hohen Druck auf das Gelenk ausüben zu können.

Der aus einem harten oder inkompressiblen Material bestehende Friktionskern 80 ist in dem Material der Pelotte 50 in seiner Lage fixiert. Der Friktionskern 80 weist eine kugelkappenförmige Oberfläche 83 auf bzw. eine Oberflächenausgestaltung, die in Ausgestaltung und Formgebung mit der Oberflächengestaltung der Pelotte 50 im Anordnungsbereich des Friktionskernes 80 entspricht.

Nach einer weiteren Ausführungsform ist das Material des aus einem Kunststoff, z.B. Silikonkautschuk, bestehenden Friktionskerns 80 mit dem Material der Pelotte 50 verschmolzen und mit dessen Formkörper unlösbar verbunden. Der Friktionskern 80 kann auch bei der Herstellung der Pelotte 50 durch Materialaushärtung eines Abschnittes, der den späteren Friktionskern bilden soll und daher eine größere Härte gegenüber dem weichen Material der Pelotte 50 aufweist, der Pelotte 50 erhalten werden. In beiden Fällen sollte als Material bevorzugterweise Silikonkautschuk verwendet werden.

Die Pelotte 50 kann auch beutelförmig ausgebildet sein. Dieser Beutel besteht dann aus weich-elastischen Kunststoffen. Der Innenraum des Beutels ist mit einem gasförmigen Medium, wie z.B. Luft, oder einem flüssigen Medium, wie z.B. einem zähflüssigen Silikonöl, Wasser od.dgl. gefüllt. Der Friktionskern 80 ist dann an der Innenwandfläche des Beutels in seiner Lage fixiert.

Die Genu-Kniegelenkbandage 10a ist mit einem in ihrer Pelotte 50 angeordneten, wie oben beschriebenen Friktionskern 80 versehen (Fig. 1 und 2), wohingegen die Patella-Kniegelenkbandage 10b mit der Pelotte 50 versehen ist, die mit oder ohne Friktionskern 80 ausgebildet sein kann (Fig.3 und 4). Die Wirkungsweise der Pelotte 50 ist die einer Druckpelotte.

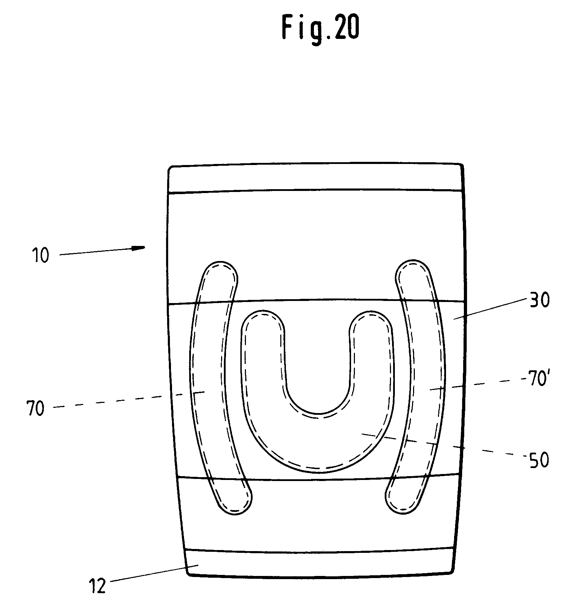

Bei dem in Fig. 3 und 4 gezeigten Ausführungsbeispiel ist seitlich der Pelotte 50 eine Pelottenstabilisierungsspange 70 angeordnet. Es besteht jedoch auch die Möglichkeit, in dem vorderen Bandagenteil 12 zu beiden Seiten der Schenkel 51,52 der Pelotte 50 je eine Pelottenstabilisierungsspange 70,70' anzuordnen (Fig.20).

Die nach einer weiteren Ausführungsform im rückwärtigen Bandagenteil 11 eingenähten Pelotten 60,60' bestehen aus dem gleichen Material wie die Pelotte 50. Neben einer ellipsenförmigen Ausgestaltung können die Pelotten 60,60' auch eine andere Formgebung aufweisen. Jede dieser beiden Pelotten 60, 60' ist als wulstförmiger Körper ausgebildet. Anstelle von zwei Pelotten 60,60' kann in das rückwärtige Bandagenteil 11 auch nur eine Pelotte 60 bzw. 60' eingenäht sein.

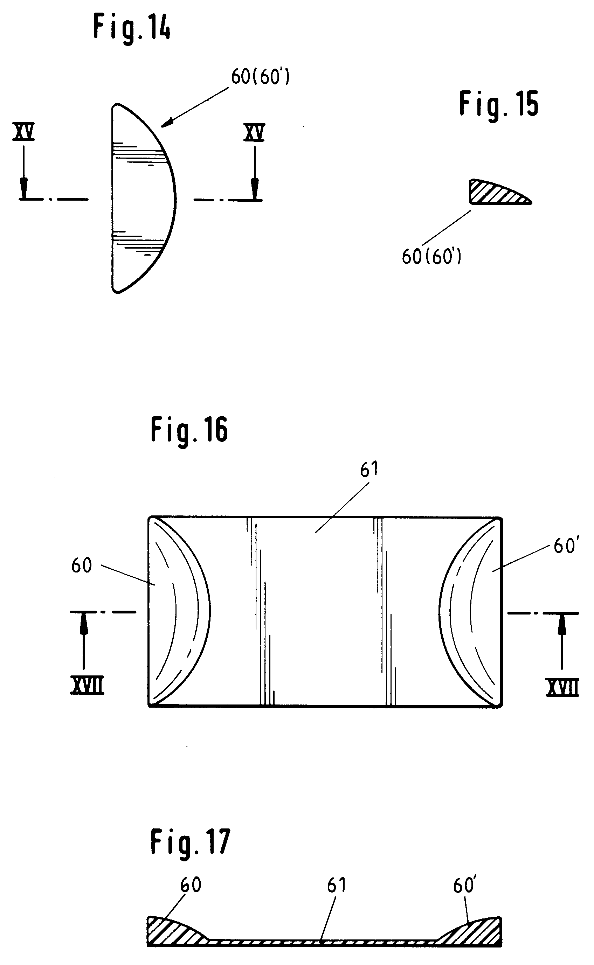

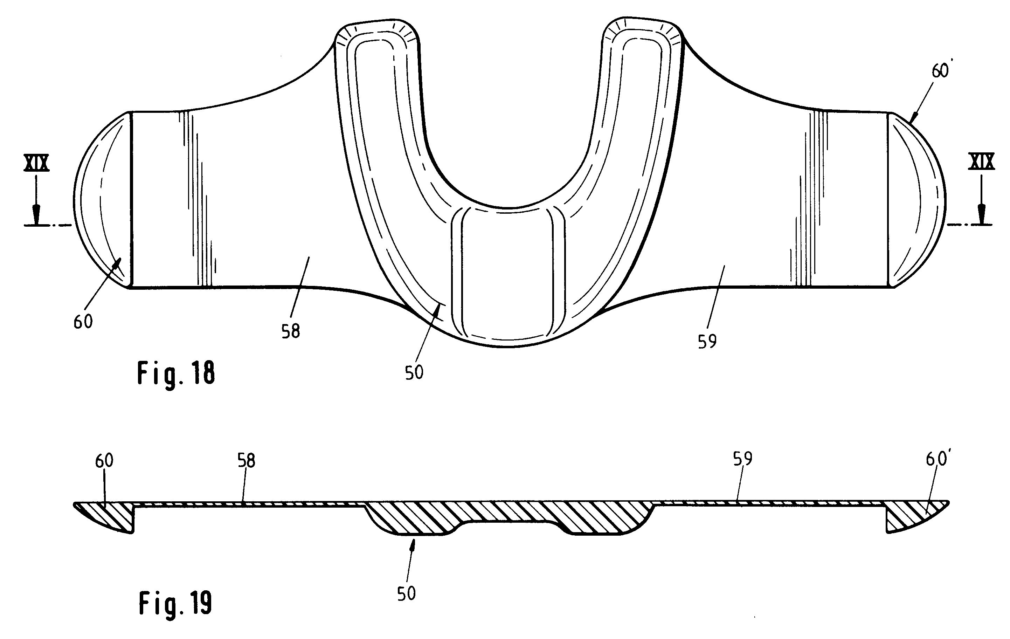

Die beiden Pelotten 60,60' in dem rückwärtigen Bandagenteil 11 können über einen Steg 61 miteinander verbunden sein (Fig.16 und 17). Es besteht darüber hinaus auch die Möglichkeit, die beiden Pelotten 60,60' in dem rückwärtigen Bandagenteil 11 über Stege 58,59 mit den Schenkeln 51,52 der Pelotte 50 zu verbinden (Fig. 18 und 19). Die die Pelotten 60,60' miteinander oder mit der Pelotte 50 verbindenden Stege 61 und 58,59 sind dünnwandig ausgebildet und bestehen aus dem gleichen Material wie die Pelotten 50, 60, 60', so daß diese Stege 61 und 58,59 eine hohe Flexibilität besitzen.

Das rückwärtige Bandagenteil 11 ist mit dem vorgespannten Wellengestrick 40 eingenäht (Fig.1 und 2). Die Pelotte 50 sowie die Pelotten 60,60' und die Pelottenstabilisierungsspange 70 sind im Bereich des in den Schlauchkörper 20 der Bandage 10 integrierten Einsatzes 30 aus dem Wellengestrick 40 angeordnet.

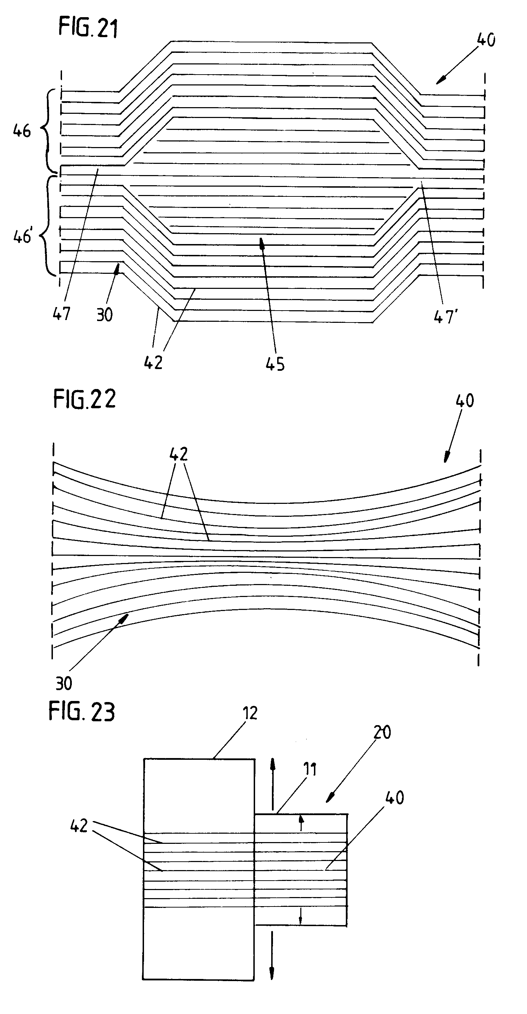

Das Wellengestrick 40 des Einsatzes 30 weist im vorderen Bandagenteil 12 eine größere Anzahl von Wellen 42 als im rückseitigen Bandagenteil 11 auf. Die Führung der Wellen 42 des Wellengestrickes 40 des Einsatzes 30 geht von einer geringeren Anzahl von Wellen im rückwärtigen Bandagenteil 11 aus zu einer Teilung der Wellen 42 in zwei im Abstand voneinander verlaufende Wellengruppen 46,46' in dem vorderen Bandagenteil 12, wobei in dem zwischen den beiden Wellengruppen 46,46' in dem vorderen Bandagenteil 12 ausgebildeten Zwischenraum 45 eine in keilförmige Endabschnitte 47,47' auslaufende Wellengruppe 46'' angeordnet ist (Fig. 21 und 22). Beim Zusammennähen des vorderen Bandagenteils 12 mit dem rückwärtigen Bandagenteil 11 wird das Wellengestrick 40 mit der geringeren Anzahl an Wellen 42 bei einem gleichzeitigen Zusammenziehen des Wellengestricks in dem vorderen Bandagenteil 12 gedehnt (Fig. 23). Eine Besonderheit der Bandage 10 besteht darin, daß für den Einsatz 30, der dort vorgesehen ist, wo die höchsten Dehnungs-Wechselbeanspruchungen der Bandage auftreten, eine besondere Textilstruktur, nämlich das Wellengestrick 40, verwendet wird, welches nachstehend näher erläutert wird. Es besteht jedoch auch die Möglichkeit, die Wellen 42 des Wellengestricks 40 des Einsatzes 30 in dem vorderen Bandagenteil 12 geradlinig und parallel verlaufen zu lassen.

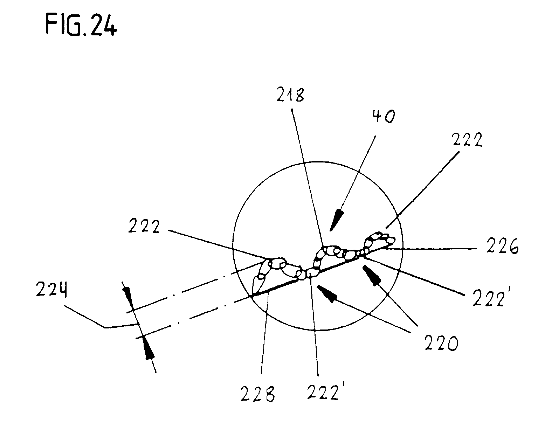

Die streifenförmige Wiedergabe des Einsatzes 30 in den Fig. 21 und 22 deutet an, daß das Wellengestrick 40 in diesem Bereich ein Relief bildet, welches in der Darstellung gemäß Fig.24 verdeutlicht wird. Bei diesem Relief handelt es sich um eine Wellenstruktur, die zumindest auf einer Seite der Bandage - bei der in den Zeichnungen gezeigten Ausführungsformen auf der dem Körper abgewandten Seite - ausgebildet wird. Dabei ist eine Reihe von Halbwellen 218 unter Zwischenschaltung von Knotenpunkten bzw. Knotenbereichen 220 aneinandergereiht, die wie folgt entstehen:

Ein von Maschen 222 gebildetes Deckgestrick 224 ist im Bereich einzelner Maschen 222' mit einer elastischen Fadenanordnung 226 auf der Unterseite des Deckgestricks 224 derart fest verbunden, daß eine Reihe von Maschen 222 des Deckgestricks 224 - bei der Ausführungsform vier - von einer längeren Masche 228 der darunterliegenden, elastischen Fadenanordnung 226 überbrückt wird. Die Maschen 222 zwischen den Knotenpunkten bzw. -bereichen 220 werden dadurch nach oben ausgebaucht, wodurch die Querwellen 218 vorgespannt und stabilisiert werden. Durch Variation der Anzahl der überbrückten Maschen 222 und/oder der Maschen 228 kann auf das Verformungsverhalten und die Dauerelastizität gezielt Einfluß genommen werden.

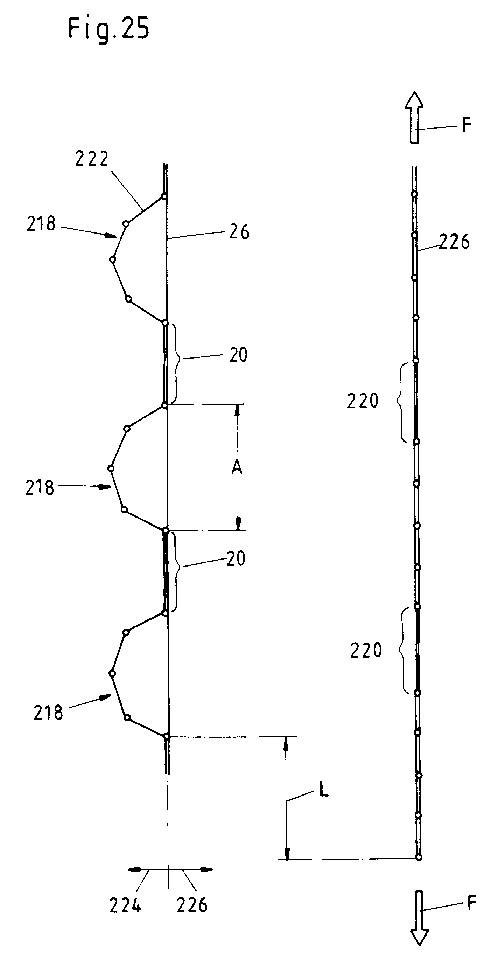

Das Verformungsverhalten des auf diese Art und Weise gebildeten Wellengestricks 40 geht im einzelnen aus der Darstellung gemäß Fig.25 hervor. Auf der linken Seite ist das Wellengestrick im entlasteten Zustand gezeigt. Die Maschen sind durch Linien angedeutet und die Verbindung der Maschen untereinander durch kleine Kreise.

Das auf der Unterseite vorgesehene elastische Garn 226 überbrückt die Knotenbereiche 220, zwischen denen jeweils vier Maschen 222 des Deckgestricks 224 ausgebildet sind.

Durch die Vorspannung der elastischen Fadenanordnung 226 werden die Maschen 222 zu Halbwellen 218 ausgebaucht, wodurch die Reliefstruktur entsteht. Die Halbwellen 218 weisen jeweils zwischen zwei und zwölf, vorzugsweise vier, Maschenreihen auf.

Auf der rechten Seite ist dargestellt, wie sich das Wellengestrick 40 bei Beanspruchung durch eine Zugkraft F verhält. Es ist erkennbar, daß die elastische Fadenanordnung 226 zwischen den Knotenbereichen 220 gedehnt wird, ohne daß eine zusätzliche Dehnungsbeanspruchung im Bereich der Halbwellen 218, d.h. im Bereich der Maschen 222, auftritt. Das Wellengestrick 40 kann sich dementsprechend um das Maß L längen, bevor die Maschen 222 des Deckgestricks 224 auf Zug beansprucht werden. Dieses Maß L schafft dementsprechend eine Dehnungsreserve des Bandagengestricks im Vergleich zu herkömmlichen textilen Flächengebilden.

Die Verbindung zwischen Deckgestrick 224 und elastischer Fadenanordnung 226 kann auf verschiedenste Art und Weise erfolgen. Es ist auch möglich, die Verbindung derart zu wählen bzw. herzustellen, daß auf beiden Seiten des Wellengestricks 40 Wellen 42 gebildet werden. Das Deckgestrick 224 braucht nicht einflächig ausgebildet zu sein. Durch die vorstehende Struktur des Wellengestricks 40 kann trotz Bereitstellung einer hohen Dehnungselastizität für das Deckgestrick normales Strickgarn, wie z.B. Baumwolle oder Polyamidgarn, verwendet werden. Für die elastische Fadenanordnung 226 wird vorzugsweise höherelastisches Garn, wie z.b. Umwindungsgarn, verwendet, wobei dieser elastische Faden zusätzlich plattiert werden kann, um die Verschleißfestigkeit des textilen Gewebes bzw. Gewirks zu verbessern.

In die Maschen 222 des Deckgestricks 224 kann ferner ein Einlegefaden eingearbeitet werden, um auch in diesem Bereich der wellenförmigen Reliefstruktur eine Kompressionswirkung der Bandage zu erreichen.

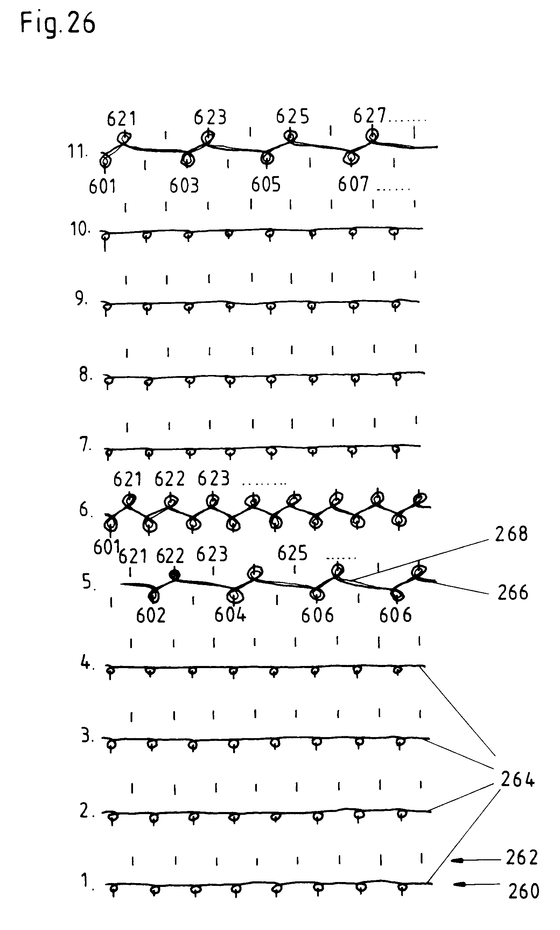

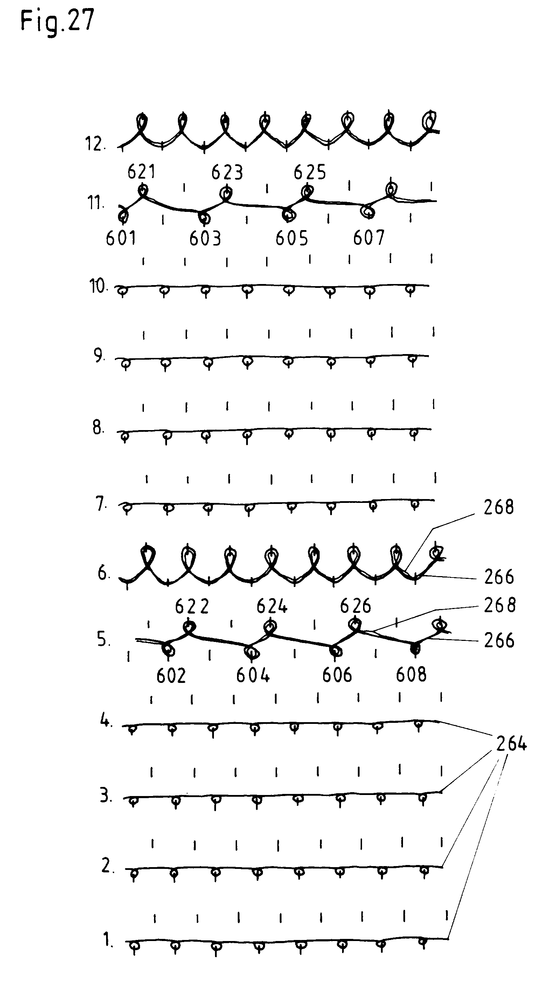

In den Fig. 26 und 27 sind zwei Möglichkeiten aufgezeigt, wie das vorstehend erläuterte Wellengestrick 40 auf automatischen Strickmaschinen hergestellt werden kann. Hierbei werden zwei Nadelbetten verwendet, nämlich ein erstes Nadelbett 260 und ein zweites Nadelbett 262 mit im gleichen Abstand zueinander angeordneten Nadeln. Gestrickt werden auf dem ersten Nadelbett 260 mehrere - im gezeigten Ausführungsbeispiel vier - Maschenreihen aus normalem Strickgarn, wie z.B. Baumwoll- oder Polyamidgarn 264. Anschließend werden zwei Maschenreihen auf beiden Nadelbetten 260,262 mit elastischem Garn, wie z.B. Gummi oder Umwindungsgarn 268 gestrickt, wobei diesem elastischen Garn vorzugsweise ein Plattierfaden 266 beigegeben wird. Bei der fünften Strickreihe wird nur auf ausgewählten Nadeln 602,604,606,... usw. bzw. 622,624,626,... usw. beider Nadelbetten gestrickt. Der Plattierfaden kann von einem Polyamid HE-Faden gebildet sein. Die Darstellung läßt erkennen, daß die Maschenreihe sechs wieder auf allen Nadeln der beiden Nadelbetten 260,262 gestrickt wird. Dies ist jedoch nicht unbedingt erforderlich.

Es folgen dann wiederum vier Maschenreihen mit gewöhnlichem Garn auf dem ersten Nadelbett 260 und abschließend zwei weitere Maschenreihen unter Verwendung elastischen Garns, wobei sich die elfte Strickreihe von der fünften Strickreihe dadurch unterscheidet, daß die beteiligten Nadeln der Nadelbetten 260,262 um eines versetzt sind.

Fig. 27 zeigt ein anderes Muster mit einer etwas anderen Bindung im Bereich der späteren Gestrick-Knoten 220. Die Strickreihen eins bis fünf und sieben bis elf entsprechen dem Strickmuster gemäß Fig.26. Unterschiedlich ist lediglich die Ausbildung der 6. und der 12 Strickreihe, so daß ein näheres Eingehen auf diese Figur nicht erforderlich erscheint.

Abweichend von dem zuvor beschriebenen Herstellungsverfahren ist es auch möglich, zunächst auf einem Nadelbett eine oder mehrere Maschenreihen aus elastischem Garn, wie z.B. Umwindegarn, zu stricken, und auf dem anderen Nadelbett Maschenreihen aus normalem Strickgarn, wie z.B. Baumwoll- oder Polyamidgarn, wobei anschließend eine oder mehrere Maschenreihen mit allen oder einzelnen Nadeln beider Nadelbetten gestrickt werden.

Es erfolgt keine Beschränkung darauf, daß der Einsatz 30 aus einem vorangehend beschriebenen Wellengestrick 40 besteht. Entscheidend ist lediglich, daß das den Einsatz 30 bildende textile Flächengebilde einen solchen Aufbau erhält, daß eine entweder eingearbeitete oder nach vorbestimmtem Muster mit einer textilen Deckstruktur verbundene elastische Faden Anordnung dem Flächengebilde eine solche innere Vorspannung gibt, daß zumindest auf einer Seite ein wellenartiges Relief entsteht, das dann durch äußere Beanspruchung zunächst glatt bzw. glatter gezogen werden kann, ohne bereits in dieser Phase die Deckstruktur auf Dehnung zu beanspruchen.

Der Einsatz 30 aus dem Wellengestrick 40 verhindert eine Faltenbildung bzw. wirkt einer Faltenbildung entgegen, insbesondere im inneren Beugebereich der angelegten Bandage 10.

Bevorzugterweise ist die Pelottenstabilisierungsspange 70 beidendseitig aus dem Einsatz 30 des Wellengestricks 40 herausgeführt, wobei die freien Spangenenden in das Gewebe oder Gewirk des Schlauchskörpers 20 der Bandage 10 eingenäht sind.

Um vom Stegbereich der Pelotte 50 einen fast stufenlosen Übergang zur Bandage zu erhalten, kann der Steg der Pelotte 50 mit einer zungenartigen, zum äußeren Rand verjüngend auslaufenden Anformung versehen sein, die aus dem Pelottenmaterial besteht. Auch besteht die Möglichkeit, den äußeren Randbereich des Steges in seinen zu beiden Seiten der Stegmitte liegenden Stegabschnitten verjüngend auslaufen zu lassen. Der äußere Rand des Pelottensteges ist somit nach außen flach auslaufend ausgebildet. Die freien Enden der beiden Schenkel der Pelotte können elefantenfußartig verbreitert ausgebildet sein, wodurch der Auflage- und Wirkungsbereich der Pelotte 50 verbessert wird.

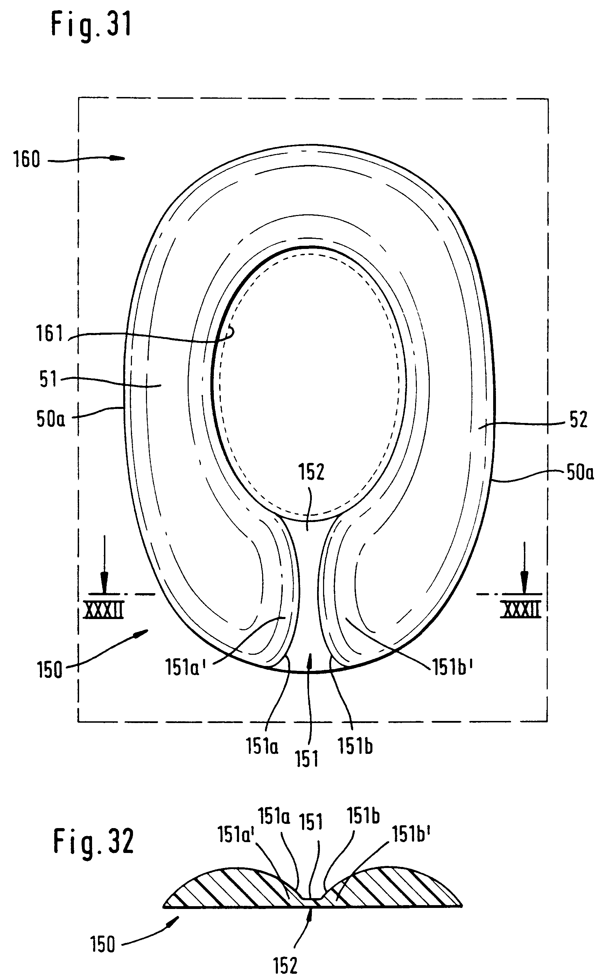

Nach einer zweiten Ausführungsform ist die im vorderen Bandagenteil 12 angeordnete Pelotte 150 ringförmig ausgebildet und weist vorzugsweise eine ovale Form bzw. die Form einer Ellipse auf. Im unteren Bereich gehen die beiden Pelottenschenkel 51,52 in einen schmalen, dünnwandigen Pelottenabschnitt 152 in Form einer stegartigen Vertiefung 151 über, wobei die die stegartige Vertiefung 151 begrenzenden seitlichen Ränder 151a,151b geradlinig oder kreisbogenförmig, wie in Fig.31 dargestellt, verlaufen, wodurch eine seitliche Friktion, d.h. eine Friktion zu beiden Seiten des Sehnenansatzes, erreicht wird. Diese Friktion wird noch dadurch unterstützt, daß die im Bereich der seitlichen Ränder 151a,151b endenden Abschnitte 151a',151b' der Pelottenschenkel 51,52 eine wulstartige Ausgestaltung aufweisen (Fig.32). Die Pelotte 150 besteht aus den gleichen Materialien wie die Pelotte 50. Die Befestigung der Pelotte 150 an dem vorderen Bandagenteil 12 erfolgt vermittels eines innenwandseitig angebrachten Gewebezuschnitts 160 aus einem dünnen Material, so daß eine Doppelwandigkeit erhalten wird. In dem so ausgebildeten Zwischenraum wird dann die Pelotte 150, die durch eine bei 161 angedeutete Naht, die den Zuschnitt 160 mit dem Gewebe des Bandagenteils 12 verbindet, in unverrückbarer Stellung gehalten (Fig.31).

## Patentansprüche

1. Bandage für das Kniegelenk aus elastischem Bandagenstoff in Schlauchform mit einem vorderen Bandagenteil (12) und einem rückwärtigen Bandagenteil (11) und mit mindestens einem eingearbeiteten, längsverlaufenden Federstab, wobei die Bandage (10a) aus einem anatomisch geformten Schlauchkörper (20) aus einem Gewebe oder Gestrick besteht und wobei in dem vorderen Bandagenteil (12) eine im Bereich über der Kniescheibe bei angelegter Bandage (10a) liegende, nach oben offene, die Quadrizeps-Sehne freilassende Pelotte (50) aus einem weichen oder weichelastischen Material angeordnet ist, dadurch gekennzeichnet, daß das Gewebe oder Gestrick der Bandage (10a) einen rundumlaufenden Einsatz (30) aus einem hochelastischen Wellengestrick (40) aufweist und daß die nach oben offene Pelotte (50) mit einem bogenförmigen Verlauf der Außenränder (50a,50b) der beiden Pelottenschenkel (51,52) und mit einer außenseitig liegenden, ebenen Grundfläche (54) sowie mit einem halbkreis- oder teilkreisförmigen Schenkelquerschnitt mittig in dem die beiden Pelottenschenkel (51,52) verbindenden Steg (53) eine innenseitig liegende Vertiefung (55) zur Anpassung an den Schienbeinkopf und an den Verlauf des Kniescheibenbandes aufweist, wobei die Länge der Vertiefung (55) der Breite des Pelottensteges (53) entspricht.

2. Bandage für das Kniegelenk aus elastischem Bandagenstoff in Schlauchform mit einem vorderen Bandagenteil (12) und einem rückwärtigen Bandagenteil (11), wobei die Bandage (10a) aus einem anatomisch geformten Schlauchkörper (20) aus einem Gewebe oder Gestrick besteht und wobei in dem vorderen Bandagenteil (12) eine im Bereich über der Kniescheibe bei angelegter Bandage (10a) liegende ringförmige Pelotte (150) aus einem weichen oder weichelastischen Material angeordnet ist, die zur Verringerung eines Druckes auf das Kniescheibenband im unteren Bereich mit einer stegartigen Vertiefung (151) unter Ausbildung eines dünnwandigen Pelottenabschnittes und mit einem bogenförmigen Verlauf der Außenränder (50a) der beiden Pelottenschenkel (51,52) und mit einer außenseitig liegenden, ebenen Grundfläche (152) versehen ist, dadurch gekennzeichnet, daß das Gewebe oder das Gestrick der Bandage (10a) einen rundumlaufenden Einsatz (30) aus einem hochelastischen Wellengestrick (40) aufweist, daß die Bandage mit mindestens einem eingearbeiteten, längsverlaufenden Federstab (13;14) versehen ist und daß die ringförmige oder ovale Pelotte (150) einen halbkreis- oder teilkreisförmigen Schenkelquerschnitt und mittig in dem die beiden Pelottenschenkel (51,52) verbindenden Steg eine innenseitig liegende Vertiefung (151) aufweist, wobei die Länge der Vertiefung (151) der Breite des Pelottensteges entspricht, wobei die seitlichen Ränder (151a, 151b) geradlinig oder kreisbogenförmig verlaufend sind und wobei die im Bereich der seitlichen Ränder (151a,151b) endenden Abschnitte (151a',151b') der Pelottenschenkel (51,52) zur Erzielung einer Friktion zu beiden Seiten des Sehnenansatzes eine wulstartige Ausgestaltung aufweisen.

3. Bandage für das Kniegelenk aus einem elastischen Bandagenstoff in Schlauchform mit einem vorderen Bandagenteil (12) und einem rückwärtigen Bandagenteil (11) und mit mindestens einem eingearbeiteten, längsverlaufenden Federstab, wobei die Bandage (10b) aus einem anatomisch geformten Schlauchkörper (20) aus einem Gewebe oder Gestrick besteht, und wobei in dem vorderen Bandagenteil (12) eine im Bereich über der Kniescheibe bei angelegter Bandage (10b) liegende, die Quadrizeps-Sehne freilassende Pelotte (50) aus einem weichen oder weichelastischen Material angeordnet ist, dadurch gekennzeichnet, daß das Gewebe oder Gestrick der Bandage (10b) einen rundumlaufenden Einsatz (30) aus einem hochelastischen Wellengestrick (40) aufweist und daß in dem vorderen Bandagenteil (12) mindestens eine eine nach oben offene oder ringförmige Pelotte (50;150) stabilisierende, in Bandagenlängsrichtung verlaufende Pelottenstabilisierungsspange (70) angeordnet ist, wobei die Pelottenstabilisierungsspange (70) integrierter Bestandteil der Pelotte (50;150) oder seitlich zu dieser liegend ist.

4. Bandage für das Kniegelenk aus elastischem Bandagenstoff in Schlauchform mit einem vorderen Bandagenteil (12) und einem rückwärtigen Bandagenteil (11) und mit mindestens einem eingearbeiteten, längsverlaufenden Federstab, wobei die Bandage (10a) aus einem anatomisch geformten Schlauchkörper (20) aus einem Gewebe oder Gestrick besteht und wobei in dem vorderen Bandagenteil (12) eine im Bereich über der Kniescheibe bei angelegter Bandage (10a) liegende erste, die Quadrizeps-Sehne freilassende Pelotte (50) aus einem weichen oder weich-elastischen Material angeordnet ist, dadurch gekennzeichnet, daß das Gewebe oder Gestrick der Bandage (10a) einen rundumlaufenden Einsatz (30) aus einem hochelastischen Wellengestrick (40) aufweist und daß in das rückwärtige Bandagenteil (11) eine zweite oder eine zweite und eine dritte, den Ansatz der ischiokruralen Muskulatur beaufschlagende Pelotte (60,60') aus einem weichen oder weich-elastischen Material eingesetzt ist.

5. Bandage für das Kniegelenk aus einem elastischen Bandagenstoff in Schlauchform mit einem vorderen Bandagenteil (12) und einem rückwärtigen Bandagenteil (11) und mit mindestens einem eingearbeiteten, längsverlaufenden Federstab, wobei die Bandage (10b) aus einem anatomisch geformten Schlauchkörper (20) aus einem Gewebe oder Gestrick besteht und wobei in dem vorderen Bandagenteil (12) eine im Bereich über der Kniescheibe bei angelegter Bandage (10b) liegende erste, die Quadrizeps-Sehne freilassende Pelotte (50) aus einem weichen oder weich-elastischen Material angeordnet ist, dadurch gekennzeichnet, daß das Gewebe oder Gestrick der Bandage (10a) einen rundumlaufenden Einsatz (30) aus einem hochelastischen Wellengestrick (40) aufweist und daß in dem vorderen Bandagenteil (12) mindestens eine, eine nach oben offene oder ringförmige Pelotte (50;150) stabilisierende, in Bandagenlängsrichtung verlaufende Pelottenstabilisierungsspange (70) angeordnet ist, wobei die Pelottenstabilisierungsspange (70) integrierter Bestandteil der ersten Pelotte (50;150) oder seitlich zu dieser liegend ist, und daß in das rückwärtige Bandagenteil (11) eine zweite oder eine zweite und eine dritte, den Ansatz der ischiokruralen Muskulatur beaufschlagende Pelotte (60,60') aus einem weichen oder weich-elastischen Material eingesetzt ist.

6. Bandage nach einem der Ansprüche 1 bis 5, dadurch gekennzeichnet, daß die erste Pelotte (50;150) der Bandage U-förmig oder V-förmig ausgebildet ist und bevorzugterweise eine hufeisenförmige Ausgestaltung mit einem bogenförmigen Verlauf der Außenränder (50a,50b) der beiden Pelottenschenkel (51,52) aufweist oder eine ringförmige oder eine ovale Form aufweist und eine außenseitig liegende, ebene Grundfläche (54), einen halbkreis- oder teilkreisförmigen Schenkelquerschnitt und in dem die Pelottenschenkel (51,52) miteinander verbindenden Steg (53) mittig eine innenseitig liegende Vertiefung (55) mit in Bandagenlängsrichtung verlaufenden seitlichen Führungen (56,57) zur Anpassung an den Schienbeinkopf und an den Verlauf des Kniescheibenbandes aufweist.

7. Bandage nach einem der Ansprüche 3 und 5, dadurch gekennzeichnet, daß die Pelottenstabilisierungsspange (70) benachbart zu einem der beiden Pelottenschenkel (51,52) der ersten Pelotte (50;150) liegend ist und eine der Form des äußeren Schenkelrandes (50a;50b) entsprechende Form aufweist.

8. Bandage nach einem der Ansprüche 3,5 und 7, dadurch gekennzeichnet, daß die Pelottenstabilisierungsspange (70) in einem Abstand von dem Pelottenschenkel (51) der beiden Pelottenschenkel (51,52) der ersten Pelotte (50;150) angeordnet ist, wobei zwischen dem Pelottenschenkel (51) der ersten Pelotte (50;150) und der Pelottenstabilisierungsspange (70) gegebenenfalls ein Gewebe- oder Gestrickabschnitt (41) angeordnet ist.

9. Bandage nach einem der Ansprüche 3,5 bis 8, dadurch gekennzeichnet, daß die Pelottenstabilisierungsspange (70) abstandslos neben dem Pelottenschenkel (51) der beiden Pelottenschenkel (51,52) der ersten Pelotte (50;150) angeordnet ist.

10. Bandage nach einem der Ansprüche 3,5 bis 8, dadurch gekennzeichnet, daß die Pelottenstabilisierungsspange (70) an dem Pelottenschenkel (51) der beiden Pelottenschenkel (51,52) der ersten Pelotte (50;150) angeformt ist.

11. Bandage nach einem der Ansprüche 3,5 bis 8, dadurch gekennzeichnet, daß die Pelottenstabilisierungsspange (70) durch einen der beiden Pelottenschenkel (51,52) der ersten Pelotte (50;150) hindurchgeführt und in das Pelottenmaterial eingebettet ist.

12. Bandage nach einem der Ansprüche 3,5 bis 8, dadurch gekennzeichnet, daß die Pelottenstabilisierungsspange (70) durch einen der beiden Pelottenschenkel (51,52) der ersten Pelotte (50;150) hindurchgeführt und in das Pelottenmaterial eingebettet ist, wobei die Pelottenstabilisierungsspange (70) beidendseitig aus dem Pelottenschenkel (51) mit einem Abschnitt (70a;70b) herausgeführt ist.

13. Bandage nach einem der Ansprüche 1 bis 12, dadurch gekennzeichnet, daß die erste Pelotte (50;150) einen knopfförmigen Friktionskern (80) enthält, der in das Pelottenmaterial eingearbeitet ist und der aus einem Material besteht, das gegenüber dem Härtegrad des Pelottenmaterials einen höheren Härtegrad aufweist.

14. Bandage nach Anspruch 13, dadurch gekennzeichnet, daß die Diffrerenz zwischen der Härte der ersten Pelotte (50;150) und der Härte des Friktionskerns (80) mindestens 10 Shore A, vorzugsweise 20 Shore A, beträgt, wobei das Material der ersten Pelotte (50;150) eine unterhalb 50 Shore A liegende Härte und das Material des Friktionskerns (80) eine oberhalb 50 Shore A liegende Härte aufweist.

15. Bandage nach einem der Ansprüche 13 bis 14, dadurch gekennzeichnet, daß der Friktionskern (80) zur Lagenfixierung in der ersten Pelotte (50;150) im Bereich seiner umlaufenden Wandfläche (81) eine rillenförmige Ausnehmung, Einziehung, wie Nut, Hinterschneidung, Verzahnung od.dgl., (82) zur Aufnahme von Pelottenmaterial aufweist.

16. Bandage nach einem der Ansprüche 13 bis 15, dadurch gekennzeichnet, daß das Material des Friktionskerns (80) mit dem Material der ersten Pelotte (50;150) verschmolzen und mit dem Pelottenmaterial unlösbar verbunden ist, wobei der Friktionskern (80) und die erste Pelotte (50;150) aus Kunststoffen, insbesondere Silikonkautschuken od.dgl., besteht.

17. Bandage nach einem der Ansprüche 14 bis 16, dadurch gekennzeichnet, daß der Friktionskern (80) bei der Herstellung der ersten Pelotte (50;150) durch Materialaushärtung eines Abschnittes der ersten Pelotte (50;150) erhalten wird, wobei der Friktionskern (80) und die erste Pelotte (50;150) aus Kunststoffen, insbesondere Silikonkautschuk od. dgl., unterschiedlicher Härtegrade besteht.

18. Bandage nach einem der Ansprüche 1 bis 17, dadurch gekennzeichnet, daß alle Pelotten (50;150;60,60') aus Filz, Moosgummi, Neopren, Gummi, einem viskoelastischen Silikonkautschuk oder einem elastischen, kompressiblen, durch Druck verformbaren Silikonkautschuk oder einem Material mit gleichen Elastizitätseigenschaften, wie Naturkautschuk, Silikonkautschuk od. dgl., besteht.

19. Bandage nach einem der Ansprüche 13 bis 18, dadurch gekennzeichnet, daß der Friktionskern (80) aus einem inkompressiblen Kunststoff, wie einem natürlichen oder künstlichen Kautschuk, Hartgummi, Chloropren-Polymerisat, einem gummielastischen, vernetzten Polyurethan, Polyamid, Metall, Holz od. dgl. besteht.

20. Bandage nach einem der Ansprüche 1 bis 19, dadurch gekennzeichnet, daß in der ersten Pelotte (50;150) eine den Friktionskern (80) aufnehmende Ausnehmung ausgebildet ist, der in der Ausnehmung lösbar mittels Preß- oder Klemmsitz gehalten ist.

21. Bandage nach einem der Ansprüche 1 bis 20, dadurch gekennzeichnet, daß in dem vorderen Bandagenteil (12) zu beiden Seiten der Pelottenschenkel (51,52) der ersten Pelotte (50;150) mindestens je eine Pelottenstabilisierungsspange (70,70') angeordnet ist.

22. Bandage nach einem der Ansprüche 4 bis 21, dadurch gekennzeichnet, daß die zweite und dritte Pelotte (60,60') in dem rückwärtigen Bandagenteil (11) über einen Steg (61) miteinander verbunden sind, wobei die zweite und dritte Pelotte (60,60') gegebenenfalls über Stege (58,59) mit den Pelottenschenkeln (51,52) der ersten Pelotte (50;150) verbunden sind.

23. Bandage nach einem der Ansprüche 1 bis 22, dadurch gekennzeichnet, daß die Federstäbe (13,14) eine bogenförmige Ausgestaltung aufweisen.

24. Bandage nach einem der Ansprüche 1 bis 23, dadurch gekennzeichnet, daß das rückwärtige Bandagenteil (11) mit vorgespanntem Strick oder Wellengestrick (40) eingenäht ist.

25. Bandage nach einem der Ansprüche 1 bis 24, dadurch gekennzeichnet, daß die erste Pelotte (50;150), die zweite und die dritte Pelotte (60,60') und die Pelottenstabilisierungsspange (70) im Bereich des Einsatzes (30) aus dem Wellengestrick (40) angeordnet sind.

26. Bandage nach einem der Ansprüche 1 bis 25, dadurch gekennzeichnet, daß die erste Pelotte (50;150), die zweite und dritte Pelotte (60,60') und die Pelottenstabilisierungsspange (70) in taschenförmigen Aufnahmen angeordnet sind, die an der Bandage (10) ausgebildet oder eingenäht sind.

27. Bandage nach einem der Ansprüche 1 bis 26, dadurch gekennzeichnet, daß das Wellengestrick (40) des Einsatzes (30) in dem vorderen Bandagenteil (12) eine größere Anzahl von Wellen (42) als in dem rückwärtigen Bandagenteil (11) aufweist.

28. Bandage nach einem der Ansprüche 1 bis 27, dadurch gekennzeichnet, daß die Wellen (42) des Wellengestricks (40) des Einsatzes (30) in dem vorderen Bandagenteil (12) geradlinig und parallel verlaufend sind.

29. Bandage nach einem der Ansprüche 1 bis 28, dadurch gekennzeichnet, daß die Führung der Wellen (42) des Wellengestricks (40) des Einsatzes (30) von einer geringen Anzahl von Wellen im rückwärtigen Bandagenteil (11) aus zu einer Teilung der Wellen (42) in zwei im Abstand voneinander verlaufende Wellengruppen ( 46,46') in dem vorderen Bandagenteil (12) erfolgt, wobei in dem zwischen den beiden Wellengruppen (46,46') eine in keilförmige Endabschnitte (47,47') auslaufende Wellengruppe (46'') angeordnet ist.

30. Bandage nach einem der Ansprüche 1 bis 29, dadurch gekennzeichnet, daß beim Zusammennähen des vorderen Bandagenteils (12) mit dem rückwärtigen Bandagenteil (11) das Wellengestrick (40) mit der geringeren Wellenanzahl in dem rückwärtigen Bandagenteil (11) das Wellengestrick in dem vorderen Bandagenteil (12) zusammengezogen wird.

31. Bandage nach einem der Ansprüche 1 bis 30, dadurch gekennzeichnet, daß über den das rückwärtige Bandagenteil (11) mit dem vorderen Bandagenteil (12) verbindenden Nähten Federstäbe (13,14) in das Schlauchkörpermaterial eingearbeitet sind.

32. Bandage nach einem der Ansprüche 3,5 bis 31, dadurch gekennzeichnet, daß die Pelottenstabilisierungsspange (70) elastisch ausgebildet ist.

33. Bandage nach einem der Ansprüche 3,5 bis 31, dadurch gekennzeichnet, daß die Pelottenstabilisierungsspange (70) federnd-elastisch und in Längsrichtung unelastisch ausgebildet ist.

34. Bandage nach einem der Ansprüche 1 bis 33, dadurch gekennzeichnet, daß die zweite und dritte Pelotte (60;60') halbellipsenförmig ausgebildet sind oder eine andere geometrische Form aufweisen.

35. Bandage nach einem der Ansprüche 1 bis 34, dadurch gekennzeichnet, daß die zweite und dritte Pelotte (60,60') zu einer Pelotte zusammengefaßt sind.

## Claims

1. Bandage for the knee joint of elastic bandage cloth in tubular form with a front bandage portion (12) and a rear bandage portion (11) and with at least one inserted, longitudinally proceeding spring bar, in which the bandage (10a) is comprised of an anatomically configured tubular body (20) of a woven fabric or a knitted fabric and wherein, in the front bandage portion (12), a pressure pad (50) of a soft or soft elastic material is disposed so as to be located in the area above the patella when the bandage (10a) is applied, which is upwardly open and which leaves the quadriceps tendon uncovered,
characterized in that
the woven or knitted fabric of the bandage (10a) possesses a circumferentially proceeding inset (30) of a highly elastic wavily knitted fabric (40) and in that the upwardly open pressure pad (50), with a curved course of the outer rims (50a,50b) of the two pressure pad legs (51,52) and with an externally located, plane basis (54) as well as with a semicircular leg cross section, centrally in the web (53) connecting the two pressure pad legs (51,52), is provided with an internally located indentation (55) for the adaptation to the head of the tibia and to the course of the patellar tendon, while the length of the indentation (55) corresponds to the width of the pressure pad web (53).

2. Bandage for the knee joint of elastic bandage cloth in tubular form with a front bandage portion (12) and a rear bandage portion (11), in which the bandage (10a) is comprised of an anatomically configured tubular body (20) of a woven or a knitted fabric and wherein, in the front bandage portion (12), a pressure pad (50) of a soft or soft-elastic material is disposed so as to be located in the area above the patella when the bandage (10a) is applied, which, for the reduction of a pressure on the patellar tendon, is, in the lower region, provided with a web-like depression (151) while forming a thin-walled pressure pad section and with a curved course of the external rims (50a) of the two pressure pad legs (51,52) and with an externally located, plane basis (152),
characterized in that
the woven or knitted fabric of the bandage (10a) possesses a circumferentially proceeding inset (30) of a highly elastic wavily knit fabric (40), in that the bandage is fitted with at least one inserted, longitudinally proceeding spring bar (13;14) and in that the annular or oval pressure pad (150) possesses a semicircular or pitch-circular cross section and, centrally, in the web connecting the two pressure pad legs (51,52), an internally located depression (151), wherein the length of the depression (151) corresponds to the width of the pressure pad web, wherein the lateral rims (151a, 151b) proceed rectilinearily or arcuately and wherein the sections (151a',151b') terminating within the region of the lateral rims (151a,151b) of the pressure pad legs (51,52) possess a bead-like configuration for achieving a friction on both sides of the tendon attachment.

3. Bandage for the knee joint of an elastic bandage cloth in tubular form with a front bandage portion (12) and a rear bandage portion (11) and with at least one inserted, longitudinally proceeding spring bar, wherein the bandage (10b) is comprised of an anatomically configured tubular body (20) of a woven or knitted fabric and wherein, in the front bandage portion (12), a pressure pad (50) of a soft or soft-elastic material is disposed so as to be located in the area above the patella when the bandage (10b) is applied so as to leave the quadriceps tendon uncovered,
characterized in that
the woven or knitted fabric of the bandage (10b) possesses a circumferentially proceeding inset (30) of a highly elastic wavily knitted fabric (40) and in that, in the front bandage portion (12), at least one pressure pad stabilization clasp (70) proceeding in the longitudinal direction of the bandage and stabilizing an upwardly open or annular pressure pad (50;150) is disposed, wherein the pressure stabilization clasp (70) is an integral component part of the pressure pad (50;150) or is located laterally to the same.

4. Bandage for the knee joint of elastic bandage cloth in tubular form with a front bandage portion (12) and a rear bandage portion (11) and with at least one inserted, longitudinally proceeding spring bar, wherein the bandage (10a) of an anatomically configured tubular body (20) is comprised of a woven or knitted fabric and wherein, in the front bandage portion (12), a first pressure pad (50) of a soft or soft-elastic material is disposed so as to be located within the region above the patella when the bandage (10a) is applied and which leaves the quadriceps tendon uncovered,
characterized in that
the woven or knitted fabric of the bandage (10a) possesses a circumferentially proceeding inset (30) of a highly elastic wavily knitted fabric (40) and in that, into the rear bandage portion (11), a second or a second and a third pressure pad (60,60') acting upon the attachment of the ischiocrural muscular system of a soft or soft-elastic material is inserted.

5. Bandage for the knee joint of an elastic bandage cloth in tubular form with a front bandage portion (12) and a rear bandage portion (11) and with at least one inserted, longitudinally proceeding spring bar, wherein the bandage (10b) is comprised of a woven or knitted fabric and wherein, in the front bandage portion (12), a pressure pad (50) of a soft or soft-elastic material is disposed so as to be located in the region above the patella when the bandage (10b) is applied and which leaves the quadriceps tendon uncovered,
characterized in that
the woven or knitted fabric of the bandage (10b) possesses a circumferentially proceeding inset (30) of a highly elastic wavily knitted fabric (40) and in that, in the front bandage portion (12), at least one pressure pad stabilization clasp (70) stabilizing an upwardly open or annular pressure pad (50;150) proceeding in the longitudinal direction of the bandage is disposed, wherein the pressure pad stabilization clasp (70) is an integrated component part of the first pressure pad (50;150) or is located laterally to the same and in that, into the rear bandage portion (11), a second or a second and a third pressure pad (60,60') acting upon the attachment of the ischiocrural muscular system or a soft or soft-elastic material is inserted.

6. Bandage according to any of Claims 1 to 6,
characterized in that
the first pressure pad (50;150) of the bandage is of a "U"-like or "V"-like configuration and, by preference, possesses a horseshoe-like configuration with a curved course of the outer rims (50a, 50b) of the two pressure pad legs (51,52) or possesses an annular or oval shape and a plane basis (54) located on the outside, a semicircular or pitch-circular leg cross section and, centrally, in the web (53) interconnecting the pressure pad lags (51,52), an internally located indentation (55) with lateral guidance means (56,57) proceeding in the longitudinal direction of the bandage for adaptation to the head of the tibia and to the course of the patellar tendon.

7. Bandage according to either Claim 3 or 5,
characterized in that
the pressure pad stabilization clasp (70) is located so as to be adjacent to one of the two pressure pad legs (51,52) of the first pressure pad (50; 150) and possesses a configuration which corresponds to the configuration of the external leg rim (50a;50b).

8. Bandage according to any of Claims 3,5 and 7,
characterized in that
the pressure pad stabilization clasp (70) is disposed at a distance from the pressure pad leg (51) of the two pressure pad legs (51,52) of the first pressure pad (50;150) while, between the pressure pad leg (51) of the first pressure pad (50;150) and the pressure pad stabilization clasp (70), if necessary, a woven or knitted fabric section (41) is disposed.

9. Bandage according to any of Claims 3,5 to 8,
characterized in that
the pressure pad stabilization clasp (70) is disposed without an intervening space next to the pressure pad leg (51) of the two pressure pad legs (51,52) of the first pressure pad (50;150).

10. Bandage according to any of Claims 3,5 to 8,
characterized in that
the pressure pad stabilization clasp (70) is formed onto the pressure pad leg (51) of the two pressure pad legs (51,52) of the first pressure pad (50;150).

11. Bandage according to any of Claims 3,5 to 8,
characterized in that
the pressure pad stabilization clasp (70) is passed through one of the two pressure pad legs (51,52) of the first pressure pad (50;150) and is embedded in the pressure pad material.

12. Bandage according to any of Claims 3,5 to 8,
characterized in that
the pressure pad stabilization clasp (70) is passed through one of the two pressure pad legs (51,52) of the first pressure pad (50;150) and embedded in the pressure pad material, while the pressure pad stabilization clasp (70) is at both ends passed out from the pressure pad leg (51) with a section (70a; 70b).

13. Bandage according to any of Claims 1 to 12,
characterized in that
the first pressure pad (50;150) comprises a button-like friction core (80) which is incorporated into the pressure pad material and which is comprised of a material that, in comparison with the degree of hardness of the pressure pad material, possesses a higher degree of hardness.

14. Bandage according to Claim 13,
characterized in that
the difference between the hardness of the first pressure pad (50;150) and the hardness of the friction core (80) is at least 10 Shore A, preferably 20 Shore A, while the material of the first pressure pad (50;150) possesses a hardness of below 50 Shore A and the material of the friction core (80) a hardness which exceeds 50 Shore A.

15. Bandage according to any of Claims 13 to 14,
characterized in that
the friction core (80), for the positional fixation in the first pressure pad (50;150), within the area of its circumferential wall area (81), is provided with a groove-like recess, indentation, such as a groove, undercut, serration or suchlike (82) for accommodating the pressure pad material.

16. Bandage according to any of Claims 13 to 15,
characterized in that
the material of the friction core (80) is fused with the material of the first pressure pad (50;150) and undetachably connected with the pressure pad material, while the friction core (80) and the first pressure pad (50;150) are comprised of plastic materials, more particularly silicone rubbers or the like.

17. Bandage according to any of Claims 14 to 16,
characterized in that
the friction core (80), in the fabrication of the first pressure pad (50;150) is obtained by means of material curing of a section of the first pressure pad (50;150), in which case the friction core (80) and the first pressure pad (50;150) are comprised of plastic materials, more especially silicone rubbers or suchlike possessing different degrees of hardness.

18. Bandage according to any of Claims 1 to 17,
characterized in that
all the pressure pads (50;150;60,60') are comprised of felt, cellular rubber, neoprene, rubber, a viscoelastic silicone rubber or an elastic, compressible, pressure-deformable silicone rubber or of a material possessing the same elasticity properties, such as natural rubber, silicone rubber or suchlike.

19. Bandage according to any of Claims 1 to 18,
characterized in that
the friction core (80) is comprised of an incompressible plastic, such as a natural or synthetic rubber, ebonite, chloroprene polymerisate, a rubber-elastic, cross-linked polyurethane, polyamide, metal, wood or suchlike.

20. Bandage according to any of Claims 1 to 19,
characterized in that,
in the first pressure pad (50;150), a recess accommodating the friction core (80) is formed which is detachably retained in the recess by means of force or press fit.

21. Bandage according to any of Claims 1 to 20,
characterized in that,
in the first bandage portion (12), on both sides of the pressure pad legs (51,52) of the first pressure pad (50;150), at least one pressure pad stabilization clasp (70,70') is disposed.

22. Bandage according to any of Claims 1 to 21,
characterized in that
the second and third pressure pad (60,60') are interconnected by means of a web (61) in the rear bandage portion (11), in which case the second and third pressure pad (60,60'), if necessary, are connected with the aid of webs (58,59) with the pressure pad legs (51,52) of the first pressure pad (50;150).

23. Bandage according to any of Claims 1 to 22,
characterized in that
the spring bars (13,14) possess a curved construction.

24. Bandage according to any of Claims 1 to 23,
characterized in that
the rear bandage portion (11) is sewn-in with pretensioned knitting or wavy knitting (40).

25. Bandage according to any of Claims 1 to 24,
characterized in that
the first pressure pad (50;150), the second and third pressure pad (60,60') and the pressure pad stabilization clasp (70) are disposed within the region of the insert (30) of the wavy knitting (40).

26. Bandage according to any of Claims 1 to 25,
characterized in that
the first pressure pad (50;150), the second and third pressure pad (60,60') and the pressure pad stabilization clasp (70) are disposed in pocketlike accommodating means, which are constructed on the bandage (10) or sewn into the latter.

27. Bandage according to any of Claims 1 to 26,
characterized in that
the wavy knitting (40) of the insert (30) in the front bandage portion (12) possesses a larger number of waves (42) than in the rear bandage portion (11).

28. Bandage according to any of Claims 1 to 27,
characterized in that
the waves (42) of the wavy knitting (40) of the inset (30) in the front bandage portion (12) proceed rectilinearily and parallelly.

29. Bandage according to any of Claims 1 to 28,
characterized in that
the guidance of the waves (42) of the wavy knitting (40) of the inset (30) is effected while starting out from a small number waves in the rear bandage portion (11) towards a division of the waves (42) into two wave groups (46,46') proceeding at a distance from each other in the front bandage portion (12) while, between the two wave groups (46,46'), a wave group (46'') running out into wedge-shaped terminal sections (47,47') is disposed.

30. Bandage according to any of Claims 1 to 29,
characterized in that,
when the front bandage portion (12) is sewn together with the rear bandage portion (11), the wavy knitting (40) possessing the smaller number of waves in the rear bandage portion (11), the wavy knitting in the front bandage portion (12) is contracted.

31. Bandage according to any of Claims 1 to 30,
characterized in that,
above the seams connecting the rear bandage portion (11) with the front bandage portion (12), spring bars (13,14) are incorporated into the tubular body material.

32. Bandage according to any of Claims 3,5, to 31,
characterized in that
the pressure pad stabilization clasp (70) is of elastic construction.

33. Bandage according to any of Claims 3,5 to 31,
characterized in that
the pressure pad stabilization pad (70) is constructed so as to be springably elastic and, in the longitudinal direction, so as to be inelastic.

34. Bandage according to any of Claims 1 to 33,
characterized in that
the second and third pressure pads (60;60') are of semielliptical construction or possess some other geometric configuration.

35. Bandage according to any of Claims 1 to 34,
characterized in that
the second and third pressure pad (60,60') are combined so as to form one pressure pad.

## Revendications

1. Bandage pour l'articulation du genou en étoffe élastique pour bandage en forme de tuyau avec une partie antérieure de bandage (12) et une partie postérieure de bandage (11) et avec au moins une baguette élastique incorporée, allant dans le sens de la longueur, le bandage (10a) étant constitué par un corps en tuyau (20) de forme anatomique en un tissu ou tricot et une pelote en une matière souple ou souple et élastique, ouverte vers le haut, laissant libre le tendon du muscle quadriceps crural, se situant dans la zone au-dessus de la rotule lorsque le bandage (10a) est appliqué, étant placée dans la partie antérieure du bandage (12), caractérisé en ce que le tissu ou tricot du bandage (10a) présente un insert (30) faisant tout le tour en un tricot ondulé (40) très élastique et que la pelote ouverte vers le haut (50) avec une allure en forme d'arc des bords extérieurs (50a, 50b) des deux montants de pelote (51, 52) et avec une surface de base (54) plane, se situant sur le côté extérieur, ainsi qu'avec une section de montant en forme de demi-cercle ou en forme de cercle partiel présente, au milieu dans l'entretoise (53) qui relie les deux montants de pelote (51, 52), un évidement (55) situé sur le côté intérieur pour s'adapter à la tête du tibia et à l'allure du ligament rotulien, la longueur de l'évidement (55) correspondant à la largeur de l'entretoise (53) de la pelote.

2. Bandage pour l'articulation du genou en étoffe élastique pour bandage en forme de tuyau avec une partie antérieure de bandage (12) et une partie postérieure de bandage (11), le bandage (10a) étant constitué par un corps en tuyau (20) de forme anatomique en un tissu ou tricot et une pelote (150) en une matière souple ou souple et élastique, de forme annulaire, se situant dans la zone au-dessus de la rotule lorsque le bandage (10a) est appliqué, étant placée dans la partie antérieure de bandage (12), pelote qui, pour réduire une pression sur le ligament de la rotule, est pourvue, dans la zone inférieure, d'un évidement du type entretoise (151) en formant une section de pelote à paroi mince et d'une allure en forme d'arc des bords extéreiurs (50a) des deux montants de pelote (51, 52) et d'une surface de base (152) plate, qui se situe sur le côté extérieur, caractérisé en ce que le tissu ou le tricot du bandage (10a) présente un insert faisant tout le tour (30) en un tricot ondulé très élastique (40), que le bandage est pourvu d'au moins une baguette élastique (13 ; 14) incorporée, allant dans le sens de la longueur et que la pelote (150) de forme annulaire ou ovale présente une section de montant en forme de demi-cercle ou en forme de cercle partiel et présente, au milieu de l'entretoise qui relie les deux montants de pelote (51, 52), un évidement (151) qui se situe sur le côté intérieur, la longueur de l'évidement (151) correspondant à la largeur de l'entretoise de la pelote, les bords latéraux (151a, 151b) étant en ligne droite ou en forme d'arc de cercle et les sections (151a', 151b') des montants de pelote (51, 52) qui se terminent dans la zone des bords latéraux (151a, 151b) présentant une configuration du type bourrelet pour obtenir une friction des deux côtés de la naissance du tendon.

3. Bandage pour l'articulation du genou en étoffe élastique pour bandage en forme de tuyau avec une partie antérieure de bandage (12) et une partie postérieure de bandage (11) et avec au moins une baguette élastique incorporée, allant dans le sens de la longueur, le bandage (10b) étant constitué par un corps en tuyau (20) de forme anatomique en un tissu ou tricot et une pelote (50) en une matière souple ou souple et élastique, laissant libre le tendon du muscle quadriceps crural, se situant dans la zone au-dessus de la rotule lorsque le bandage (10b) est appliqué, étant placée dans la partie antérieure du bandage (12), caractérisé en ce que le tissu ou tricot du bandage (10b) présente un insert (30) faisant tout le tour en un tricot ondulé (40) très élastique et qu'au moins une agrafe de stabilisation de pelote (70) allant dans le sens longitudinal du bandage, qui stabilise une pelote (50 ; 150) ouverte vers le haut ou de forme annulaire est placée dans la partie antérieure de bandage (12) est placée dans la partie antérieure du bandage (12), l'agrafe de stabilisation de pelote (70) étant une partie constituante intégrée de la pelote (50 ; 150) ou étant couchée latéralement par rapport à celle-ci.

4. Bandage pour l'articulation du genou en étoffe élastique pour bandage en forme de tuyau avec une partie antérieure de bandage (12) et une partie postérieure de bandage (11) et avec au moins une baguette élastique incorporée, allant dans le sens de la longueur, le bandage (10a) étant constitué par un corps en tuyau (20) de forme anatomique en un tissu ou tricot et une première pelote (50) en une matière souple ou souple et élastique, laissant libre le tendon du muscle quadriceps crural, se situant dans la zone au-dessus de la rotule lorsque le bandage (10a) est appliqué, étant placée dans la partie antérieure du bandage (12), caractérisé en ce que le tissu ou tricot du bandage (10a) présente un insert (30) faisant tout le tour en un tricot ondulé (40) très élastique et qu'une seconde ou une seconde et une troisième pelote (60, 60') en une matière souple ou souple et élastique ayant de l'impact sur la naissance de l'ensemble des muscles ischiocruraux est(sont) mise(s) en place dans la partie postérieure du bandage (11).

5. Bandage pour l'articulation du genou en étoffe élastique pour bandage en forme de tuyau avec une partie antérieure de bandage (12) et une partie postérieure de bandage (11) et avec au moins une baguette élastique incorporée, allant dans le sens de la longueur, le bandage (10b) étant constitué par un corps en tuyau (20) de forme anatomique en un tissu ou tricot et une pelote (50) en une matière souple ou souple et élastique, laissant libre le tendon du muscle quadriceps crural, se situant dans la zone au-dessus de la rotule lorsque le bandage (10b) est appliqué, étant placée dans la partie antérieure du bandage (12), caractérisé en ce que le tissu ou tricot du bandage (10b) présente un insert (30) faisant tout le tour en un tricot ondulé (40) très élastique et qu'au moins une agrafe de stabilisation de pelote (70) allant dans le sens longitudinal du bandage, qui stabilise une pelote (50 ; 150) ouverte vers le haut ou de forme annulaire est placée dans la partie antérieure de bandage (12), l'agrafe de stabilisation de pelote (70) étant une partie constituante intégrée de la première pelote (50 ; 150) ou étant couchée latéralement par rapport à celle-ci et qu'une seconde ou une seconde et une troisième pelote (60, 60') en une matière souple ou souple et élastique ayant de l'impact sur la naissance de l'ensemble des muscles ischiocruraux est(sont) placée(s) dans la partie postérieure du bandage (11).

6. Bandage selon l'une des revendications 1 à 5, caractérisé en ce que la première pelote (50 ; 150) du bandage est configurée en forme d'U ou en forme de V et présente, de préférence, une configuration en forme de fer à cheval avec une allure arquée des bords extérieurs (50a, 50b) des deux montants de la pelote (51, 52) ou présente une forme annulaire ou une forme ovale et présente une surface de base (54) plate, se situant sur le côté extérieur, une section de montant en forme de demi-cercle ou de demi-cercle partiel et, au milieu de l'entretoise (53) qui relie les montants de pelote (51, 52) l'un à l'autre, un évidement (55) qui se situe sur le côté intérieur avec des guides latéraux (56, 57) qui vont dans le sens longitudinal du bandage pour s'adapter à la tête du tibia et à l'allure du ligament rotulien.

7. Bandage selon l'une des revendicatioons 3 et 5, caractérisé en ce que l'agrafe de stabilisation de la pelote (70) est située au voisinage de l'un des deux montants de pelote (51, 52) de la première pelote (50 ; 150) et présente une forme qui correspond à la forme du bord extérieur du montant (50a ; 50b).

8. Bandage selon l'une des revendications 3, 5 et 7, caractérisé en ce que l'agrafe de stabilisation de la pelote (70) est placée à un certain écart du montant de la pelote (51) des deux montants de pelote (51, 52) de la première pelote (50 ; 150), un troncon de tissu ou de tricot (41) étant éventuellement placé entre le montant de pelote (51) de la première pelote (50 ; 150) et l'agrafe de stabilisation de la pelote (70).

9. Bandage selon l'une des revendications 3, 5 à 8, caractérisé en ce que l'agrafe de stabilisation de la pelote (70) est placée sans écart près du montant de pelote (51) des deux montants de pelote (51, 52) de la première pelote (50 ; 150).

10. Bandage selon l'une des revendications 3, 5 à 8, caractérisé en ce que l'agrafe de stabilisation de la pelote (70) est moulée sur le montant de pelote (51) des deux montants de pelote (51, 52) de la première pelote (50 ; 150).

11. Bandage selon l'une des revendications 3, 5 à 8, caractérisé en ce que l'agrafe de stabilisation de la pelote (70) traverse l'un des deux montants de pelote (51, 52) de la première pelote (50 ; 150) et est encastrée dans la matière de la pelote.

12. Bandage selon l'une des revendications 3, 5 à 8, caractérisé en ce que l'agrafe de stabilisation de la pelote (70) traverse l'un des deux montants de pelote (51, 52) de la première pelote (50 ; 150) et est encastrée dans la matière de la pelote, l'agrafe de stabilisation de la pelote (70) sortant du côté des deux extrémités du montant de la pelote (51) avec un tronçon (70a ; 70b).

13. Bandage selon l'une des revendications 1 à 12, caractérisé en ce que la première pelote (50 ; 150) contient un noyau de friction en forme de bouton (80) qui est incorporé à la matière de la pelote et qui est constitué en une matière qui présente, par rapport au degré de dureté de la matière de la pelote, un degré de dureté plus élevé.

14. Bandage selon la revendication 13, caractérisé en ce que la différence entre la dureté de la première pelote (50 ; 150) et la dureté du noyau de friction (80) est d'au moins 10 Shore A, de préférence 20 Shore A, la matière de la première pelote (50 ; 150) présentant une dureté située au-dessous de 50 Shore A et la matière du noyau de friction (80) une dureté située au-dessus de 50 Shore A.

15. Bandage selon l'une des revendications 13 à 14, caractérisé en ce que le noyau de friction (80) présente, pour fixer la position dans la première pelote (50 ; 150) dans la zone de sa surface de paroi périphérique (81), un évidement, un retrait en forme de cannelure, comme une rainure, une dépouille, une denture ou équivalent (82) pour loger la matière de la pelote.

16. Bandage selon l'une des revendications 13 à 15, caractérisé en ce que la matière du noyau de friction (80) est fondu avec la matière de la première pelote (50 ; 150) et est liée de manière indissoluble à la matière de la pelote, le noyau de friction (80) et la première pelote (50 ; 150) étant constitués par des matières synthétiques, en particulier des caoutchoucs au silicone ou équivalent.

17. Bandage selon l'une des revendications 14 à 16, caractérisé en ce que le noyau de friction (80) est obtenu, lors de la fabrication de la première pelote (50 ; 150) par durcissement de la matière d'un tronçon de la première pelote (50 ; 150), le noyau de friction (80) et la première pelote (50 ; 150) étant constitués par des matières synthétiques, en particulier du caoutchouc au silicone ou équivalent, de différents degrés de dureté.

18. Bandage selon l'une des revendications 1 à 17, caractérisé en ce que toutes les pelotes (50; 150 ; 60, 60') sont constituées en feutre, caoutchouc cellulaire, néoprène, caoutchouc, en un caoutchouc au silicone visco-élastique ou en un caoutchouc au silicone élastique, compressible, déformable par pression ou une matière aux mêmes propriétés d'élasticité, comme le caoutchouc naturel, le caoutchouc au silicone ou équivalent.

19. Bandage selon l'une des revendications 13 à 18, caractérisé en ce que le noyau de friction (80) est constitué en une matière synthétique incompressible, comme un caoutchouc naturel ou synthétique, un caoutchouc dur, un polymère au chloroprène, un polyuréthane réticulé, élastique comme du caoutchouc, du polyamide, un métal, du bois ou équivalent.

20. Bandage selon l'une des revendications 1 à 19, caractérisé en ce qu'un évidement qui loge le noyau de friction (80) est configuré dans la première pelote (50 ; 150), noyau de frictioon qui est maintenu dans l'évidement de manière amovible par ajustement par pression ou par coïncement.

21. Bandage selon l'une des revendications 1 à 20, caractérisé en ce qu'au moins une agrafe de stabilisation de pelote (70, 70') est placée dans la partie antérieure de bandage (12) respectivement sur chacun des deux côtés des montants de pelote (51, 52) de la première pelote (50 ; 150).

22. Bandage selon l'une des revendications 4 à 21, caractérisé en ce que la seconde et la troisième pelote (60, 60') sont reliées l'une à l'autre dans la partie postérieure du bandage (11) par une entretoise (61), la seconde et la troisième pelote (60, 60') étant reliées aux montants de pelote (51, 52) de la première pelote (50 ; 150), éventuellement par des entretoises (58, 59).

23. Bandage selon l'une des revendications 1 à 22, caractérisé en ce que les baguettes élastiques (13, 14) présentent une configuration en forme d'arc.

24. Bandage selon l'une des revendications 1 à 23, caractérisé en ce que la partie postérieure du bandage (11) est cousue avec un tricot ou un tricot ondulé (40) précontraint.

25. Bandage selon l'une des revendications 1 à 24, caractérisé en ce que la première pelote (50 ; 150), la seconde et la troisième pelote (60, 60') et l'agrafe de stabilisation de pelote (70) sont placées dans la zone de l'insert (30) provenant du tricot ondulé (40).

26. Bandage selon l'une des revendications 1 à 25, caractérisé en ce que la première pelote (50 ; 150), la seconde et la troisième pelote (60, 60') et l'agrafe de stabilisation de pelote (70) sont placées dans des logements en forme de poche qui sont configurés ou cousus sur le bandage (10).

27. Bandage selon l'une des revendications 1 à 26, caractérisé en ce que le tricot ondulé (40) de l'insert (30) présente, dans la partie antérieure de bandage (12), un nombre assez grand d'ondulations (42) comme dans la partie postérieure de bandage (11).

28. Bandage selon l'une des revendications 1 à 27, caractérisé en ce que les ondulations (42) du tricot ondulé (40) de l'insert (30) sont dans la partie antérieure du bandage (12) en ligne droite et sont parallèles.

29. Bandage selon l'une des revendications 1 à 28, caractérisé en ce que le guidage des ondulations (42) du tricot ondulé (40) de l'insert (30) se fait à partir d'un petit nombre d'ondulations dans la partie postérieure du bandage (11) vers une séparation des ondulations (42) en deux groupes d'ondulations (46, 46') écartés l'un de l'autre dans la partie antérieure de bandage (12), un groupe d'ondulations (46'') se terminant en sections d'extrémité en forme de clavettes (47, 47') étant placé entre les deux groupes d'ondulations (46, 46').

30. Bandage selon l'une des revendications 1 à 29, caractérisé en ce que, lorsque l'on coud ensemble la partie antérieure de bandage (12) avec la partie postérieure de bandage (11), le tricot ondulé (40) avec le nombre plus faible d'ondulations dans la partie postérieure de bandage (11) est serré avec le tricot ondulé dans la partie antérieure de bandage (12).

31. Bandage selon l'une des revendications 1 à 30, caractérisé en ce que des baguettes élastiques (13, 14) sont incorporées à la matière du corps en tuyau par les coutures qui relient la partie postérieure de bandage (11) à la partie antérieure de bandage (12).

32. Bandage selon l'une des revendications 3, 5 à 31, caractérisé en ce que l'agrafe de stabilisation de pelote (70) est configurée élastique.

33. Bandage selon l'une des revendications 3, 5 à 31, caractérisé en ce que l'agrafe de stabilisation de pelote (70) est configurée élastique comme un ressort et non élastique dans le sens de la longueur.

34. Bandage selon l'une des revendications 1 à 33, caractérisé en ce que la seconde et la troisième pelote (60 ; 60') sont configurées en forme de demi-ellipse ou présentent une autre forme géométrique.

35. Bandage selon l'une des revendications 1 à 34, caractérisé en ce que la seconde et la troisième pelote (60, 60') sont réunies en une pelote.
